(19) Europäisches Patentamt
European Patent Office
Office européen des brevets

(11) EP 3 189 030 B1

(12) EUROPÄISCHE PATENTSCHRIFT

(45) Veröffentlichungstag und Bekanntmachung des Hinweises auf die Patenterteilung:
**01.05.2019 Patentblatt 2019/18**

(21) Anmeldenummer: **15741993.8**

(22) Anmeldetag: **23.07.2015**

(51) Int Cl.:
*C07C 303/40* (2006.01) *C07C 231/12* (2006.01)
*C07C 233/43* (2006.01) *C07C 311/13* (2006.01)
*C25B 3/10* (2006.01) *C07C 213/02* (2006.01)
*C07C 217/84* (2006.01) *C25B 9/06* (2006.01)

(86) Internationale Anmeldenummer:
**PCT/EP2015/066822**

(87) Internationale Veröffentlichungsnummer:
**WO 2016/034327 (10.03.2016 Gazette 2016/10)**

(54) **2,2 -DIAMINOBIARYLE MIT ZWEI SEKUNDÄREN AMINEN UND DEREN HERSTELLUNG DURCH ELEKTROCHEMISCHE KUPPLUNG**

2,2’ -DIAMINO BIARYLS WITH TWO SECONDARY AMINES AND PRODUCTION THEREOF BY ELECTROCHEMICAL COUPLING

2,2-DIAMINOBIARYLES COMPRENANT DEUX AMINES SECONDAIRES ET PRÉPARATION DESDITS 2,2-DIAMINOBIARYLES PAR COUPLAGE ÉLECTROCHIMIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.09.2014 DE 102014217537**

(43) Veröffentlichungstag der Anmeldung:
**12.07.2017 Patentblatt 2017/28**

(73) Patentinhaber: **Evonik Degussa GmbH**
**45128 Essen (DE)**

(72) Erfinder:
- **DYBALLA, Katrin Marie**
  **45657 Recklinghausen (DE)**
- **FRANKE, Robert**
  **45772 Marl (DE)**
- **FRIDAG, Dirk**
  **45721 Haltern am See (DE)**
- **WALDVOGEL, Siegfried R.**
  **55435 Gau-Algesheim (DE)**
- **ELSLER, Bernd**
  **53127 Bonn (DE)**
- **ENDERS, Mathias**
  **55546 Hackenheim (DE)**

(56) Entgegenhaltungen:
**US-A- 4 528 076**

- **H. SAYO ET AL.: "Anodic oxidation of sulfonamides. II. Anodic oxidation of 4’-substituted benzenesulfoanilides in acetonitrile", CHEMICAL AND PHARMACEUTICAL BULLETIN, Bd. 25, Nr. 4, 1977, Seiten 640-646, XP002744597,**
- **JOAN M. INSOLE: "The circular dichroism of some 2,2?-bridged biphenyls. The absolute configuration of (+)-6,7-diphenyldibenzo[e,g][1,4]diazocine -3,10-dicarboxylic acid", JOURNAL OF THE CHEMICAL SOCIETY C: ORGANIC, 1. Januar 1971 (1971-01-01), Seite 1712, XP55213521, ISSN: 0022-4952, DOI: 10.1039/j39710001712**
- **A. E. S. FAIRFULL ET AL: "918. Some derivatives of 1 : 6-diazapyrene and 4 : 5?6 : 7-dibenzo-1 : 3-diazacyclohepta-2 : 4 : 6-triene", JOURNAL OF THE CHEMICAL SOCIETY (RESUMED), 1. Januar 1952 (1952-01-01), Seite 4700, XP55213810, ISSN: 0368-1769, DOI: 10.1039/jr9520004700**
- **KI-HYE JUNG ET AL: "Gd Complexes of DO3A-(Biphenyl-2,2’-bisamides) Conjugates as MRI Blood-Pool Contrast Agents", ACS MEDICINAL CHEMISTRY LETTERS, Bd. 3, Nr. 12, 13. Dezember 2012 (2012-12-13), Seiten 1003-1007, XP55213742, ISSN: 1948-5875, DOI: 10.1021/ml300223b**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **Ki-Hye Jung ET AL: "Supporting Information Gd-Complexes of DO3A-(Biphenyl-2,2'-bisamides) Conjugates as MRI Blood-Pool Contrast Agents", , 26. Oktober 2012 (2012-10-26), XP55214000, Gefunden im Internet: URL:http://pubs.acs.org/doi/suppl/10.1021/ml300223b/suppl_file/ml300223b_si_001.pdf [gefunden am 2015-09-17]**

## Beschreibung

Die Erfindung betrifft ein elektrochemisches Verfahren zur Herstellung von 2,2'-Diaminobiarylen.

Die direkte elektrochemische C,C-Kreuzkupplung unterschiedlich geschützter Aniline ist bisher nicht bekannt. Durch eine kupferkatalysierte C,C-Kupplung ist es zwar möglich, unsymmetrische 2,2'-Diaminobiaryle darzustellen, diese sind jedoch entweder ungeschützt (M. Smrcina, S. Vyskocil, B. Maca, M. Polasek, T. A. Claxton, A. P. Abbott, P. Kocovsky, J. Org. Chem. 1994, 59, 2156-2163.) oder beide Aminogruppen tragen die gleiche Schutzgruppe (J.-F. Cui, H. Huang, H. Wong, Synlett 2011, 7, 1018-1022. und W. Kalk, H.-S. Bien, K.-H. Schündehütte, Justus Liebigs Ann. Chem. 1977, 329-337.).

Ebenfalls ist lediglich die Synthese symmetrischer geschützter 2,2'-Diaminobiaryle mit der gleichen Schutzgruppe durch eine Ullmann-ähnliche Reaktionsführung bekannt (W. Kalk, H.-S. Bien, K.-H. Schündehütte, Justus Liebigs Ann. Chem. 1977, 329-337. und S. Zhang, D. Zhang, L. S. Liebeskind, J. Org. Chem. 1997, 62, 2312-2313.). Die Auswahl an verwendeten Schutzgruppen und Substitutionsmuster der zu kuppelnden Substanzen ist hierüber stark eingeschränkt.

Die Synthese unsymmetrischer 2,2'-Diaminobiaryle ist ferner durch eine [3,3]-sigmatrope Umlagerung möglich, welche aber schlechte Selektivitäten aufweist. Hierüber ist nur ein Zugang zu einseitig geschützten 2,2'-Diaminobiarylen mit stark begrenzter Schutzgruppenwahl gegeben.

Siehe:

- Y.-K. Lim, J.-W. Jung, H. Lee, C.-G. Cho, J. Org. Chem. 2004, 69, 5778-5781;
- S.-E. Suh, I.-K. Park, B.-Y. Lim, C.-G. Cho, Eur. J. Org. Chem. 2011, 3, 455-457;
- B.-Y. Lim, M.-K. Choi, C.-G. Cho, Tetrahedron Letters 2011, 52, 6015-6017.

Zur Darstellung unterschiedlich doppelt geschützter 2,2'-Diaminonaphthyle, wurden ungeschützte 2,2'-Diaminobiaryle zuerst einfach geschützt, um im zweiten Syntheseschritt eine zweite Schutzgruppe einzuführen (M. Shi, W.-L. Duan, G.-B. Rong, Chirality 2004, 16, 642-651; siehe auch J. M. Insole, J.Chem. Soc. C, 1971, 1712-1715). In diesem Fall erfolgt die Schützung also erst nach der Kupplung. Dies hat zur Folge, dass nur bestimmte Kombinationen von Schutzgruppen toleriert werden, da die zweite Gruppe nur unter solchen Bedingungen eingeführt werden kann unter denen die erste Gruppe stabil ist. Dadurch werden nur wenige Kombinationen toleriert. Weiterhin zeichnet sich dieses Vorgehen durch fehlende Selektivität, Aufreinigungsprozesse zwischen den Syntheseschritten und kleine Ausbeuten aus, was diese bekannte Methode nicht konkurrenzfähig macht. Weiterhin sind zum Teil eine Vielzahl an Syntheseschritten von schützen, entschützen und erneut schützen notwendig, um zu der gewünschten Zielverbindung zu kommen.

Der Erfindung lag die Aufgabe zugrunde ein Verfahren zu entwickeln, mit welchem 2,2'-Diaminobiaryle in guter Ausbeute hergestellt werden können. Insbesondere soll sich das Verfahren in vorteilhafter Weise von den aus dem Stand der Technik bekannten Herstellungsverfahren abheben.

Offenbart ist eine Verbindung welche eine der allgemeinen Strukturen (**I**) bis (**III**) aufweist:

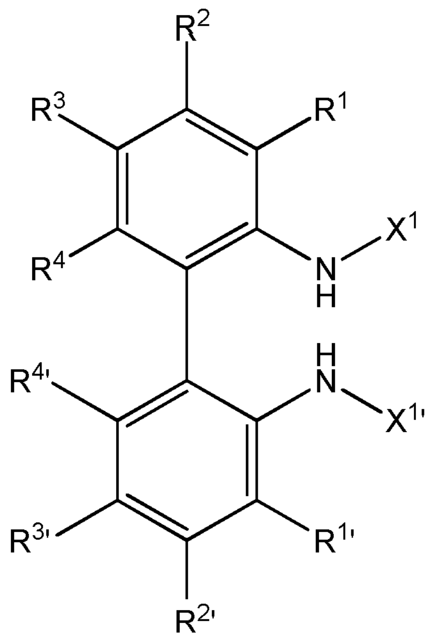

(I)

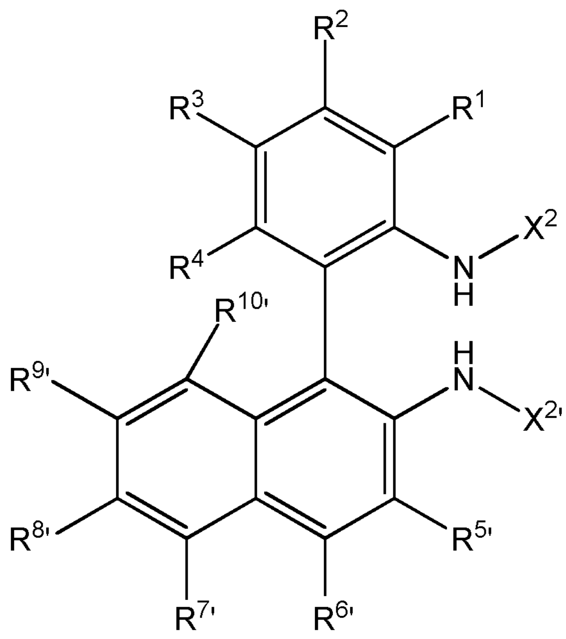

(II)

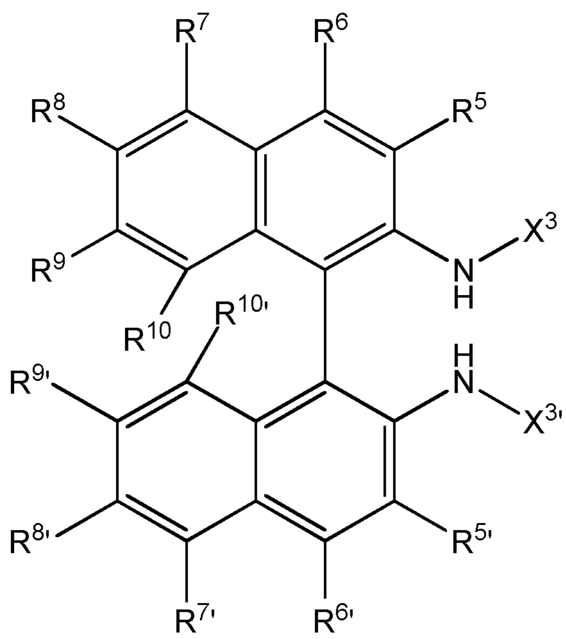

(III)

wobei

$R^1$, $R^2$, $R^3$, $R^4$, $R^{1'}$, $R^{2'}$, $R^{3'}$, $R^{4'}$ ausgewählt sind aus:
-H, $-(C_1-C_{12})$-Alkyl, $-O-(C_1-C_{12})$-Alkyl, $-O-(C_6-C_{20})$-Aryl, $-(C_6-C_{20})$-Aryl, -S-Alkyl, -S-Aryl, Halogen, $-COO-(C_1-C_{12})$-Alkyl, $-CONH-(C_1-C_{12})$-Alkyl, $-CO-(C_1-C_{12})$-Alkyl, $-CO-(C_6-C_{20})$-Aryl, -COOH, -OH, $-SO_3H$, -CN, $-N[(C_1-C_{12})\text{-Alkyl}]_2$;
$R^5$, $R^6$, $R^7$, $R^8$, $R^9$, $R^{10}$, $R^{5'}$, $R^{6'}$, $R^{7'}$, $R^{8'}$, $R^{9'}$, $R^{10'}$ ausgewählt sind aus:
-H, $-(C_1-C_{12})$-Alkyl, $-O-(C_1-C_{12})$-Alkyl, $-O-(C_6-C_{20})$-Aryl, $-(C_6-C_{20})$-Aryl, -S-Alkyl, -S-Aryl, Halogen, $-COO-(C_1-C_{12})$-Alkyl, $-CONH-(C_1-C_{12})$-Alkyl, $-CO-(C_1-C_{12})$-Alkyl, $-CO-(C_6-C_{20})$-Aryl, -COOH, -OH, $-SO_3H$, $-N[(C_1-C_{12})\text{-Alkyl}]_2$;
wobei die genannten Alkyl- und Arylgruppen substituiert sein können;
$X^1$ und $X^{1'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^1$ nicht für den gleichen Rest steht wie $X^{1'}$;
und die beiden folgenden Restepaarungen ausgeschlossen sind:

$X^1$ = Acetyl und $X^{1'}$ = Benzoyl,
$X^1$ = Benzoyl und $X^{1'}$ = Acetyl;

$X^2$ und $X^{2'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^2$ nicht für den gleichen Rest steht wie $X^{2'}$;

$X^3$ und $X^{3'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^3$ nicht für den gleichen Rest steht wie $X^{3'}$.

$-(C_1-C_{12})$-Alkyl und $-O-(C_1-C_{12})$-Alkyl können jeweils unsubstituiert oder durch einen oder mehrere gleiche

oder verschiedene Reste substituiert sein, die ausgewählt sind unter -($C_3$-$C_{12}$)-Cycloalkyl, -($C_3$-$C_{12}$)-Heterocycloalkyl, -($C_6$-$C_{20}$)-Aryl, Fluor, Chlor, Cyano, Formyl, Acyl oder Alkoxycarbonyl.

-($C_6$-$C_{20}$)-Aryl und -($C_6$-$C_{20}$)-Aryl-($C_6$-$C_{20}$)-Aryl- können jeweils unsubstituiert oder durch einen oder mehrere gleiche oder verschiedene Reste substituiert sein, die ausgewählt sind unter -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen (wie Cl, F, Br, I),-COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3$H; -$SO_3$Na, -$NO_2$, -CN, -N[($C_1$-$C_{12}$)-Alkyl]$_2$.

Im Rahmen der Erfindung umfasst der Ausdruck -($C_1$-$C_{12}$)-Alkyl geradkettige und verzweigte Alkylgruppen. Vorzugsweise handelt es sich dabei um unsubstituierte geradkettige oder verzweigte -($C_1$-$C_8$)-Alkyl- und ganz bevorzugt -($C_1$-$C_6$)-Alkylgruppen. Beispiele für -($C_1$-$C_{12}$)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -($C_1$-$C_{12}$)-Alkyl gelten auch für die Alkylgruppen in -O-($C_1$-$C_{12}$)-Alkyl, also in -($C_1$-$C_{12}$)-Alkoxy. Vorzugsweise handelt es sich dabei um unsubstituierte geradkettige oder verzweigte -($C_1$-$C_6$)-Alkoxygruppen.

Substituierte -($C_1$-$C_{12}$)-Alkylgruppen und substituierte -($C_1$-$C_{12}$)-Alkoxygruppen können in Abhängigkeit von ihrer Kettenlänge, einen oder mehrere Substituenten aufweisen. Die Substituenten sind vorzugsweise unabhängig voneinander ausgewählt unter -($C_3$-$C_{12}$)-Cycloalkyl, -($C_3$-$C_{12}$)-Heterocycloalkyl, -($C_6$-$C_{20}$)-Aryl, Fluor, Chlor, Cyano, Formyl, Acyl oder Alkoxycarbonyl.

Der Ausdruck -($C_3$-$C_{12}$)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl. Ein Beispiel für ein substituiertes Cycloalkyl wäre Menthyl.

Der Ausdruck -($C_3$-$C_{12}$)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -($C_3$-$C_{12}$)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -($C_3$-$C_{12}$)-Heterocycloalkylgruppen Tetrahydrothiophenyl, Tetrhydrofuryl, Tetrahydropyranyl und Dioxanyl.

Der Ausdruck -($C_6$-$C_{20}$)-Aryl und -($C_6$-$C_{20}$)-Aryl-($C_6$-$C_{20}$)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -($C_6$-$C_{10}$)-Aryl und -($C_6$-$C_{10}$)-Aryl-($C_6$-$C_{10}$)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -($C_6$-$C_{20}$)-Arylgruppen und -($C_6$-$C_{20}$)-Aryl-($C_6$-$C_{20}$)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen (wie Cl, F, Br, I),-COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3$H; -$SO_3$Na, -$NO_2$, -CN, -N[($C_1$-$C_{12}$)-Alkyl]$_2$. Substituierte -($C_6$-$C_{20}$)-Arylgruppen und -($C_6$-$C_{20}$)-Aryl-($C_6$-$C_{20}$)-Arylgruppen sind vorzugsweise substituierte -($C_6$-$C_{10}$)-Arylgruppen und -($C_6$-$C_{10}$)-Aryl-($C_6$-$C_{10}$)-Arylgruppen, insbesondere substituiertes Phenyl oder substituiertes Naphthyl oder substituiertes Anthracenyl. Substituierte-($C_6$-$C_{20}$)-Arylgruppen tragen vorzugsweise eine oder mehrere z.B. 1, 2, 3, 4 oder 5 Substituenten, ausgewählt unter -($C_1$-$C_{12}$)-Alkylgruppen, -($C_1$-$C_{12}$)-Alkoxygruppen.

Der Ausdruck Halogene umfasst Cl, F, Br, I, vorzugsweise Cl, Br, I,

Unter Sulfonylgruppen versteht man folgende Gruppen:

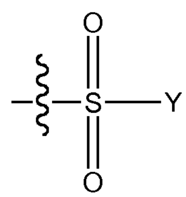

mit Y = OH, Halogene, Alkyl, Aryl, Cycloalkyl, wobei die Reste die oben genannten Definitionen beinhalten und auch substituiert sein können.

Unter Sulfenylgruppen versteht man folgende Gruppen:

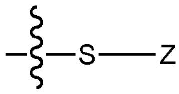

mit Z = OH, Halogene, Alkyl, Aryl, Cycloalkyl mit Z ≠ H, wobei die Reste die oben genannten Definitionen beinhalten und auch substituiert sein können.

In einer nicht erfindungsgemäßen Ausführungsform sind $X^1$ und $X^{1'}$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform ist $X^1$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform ist $X^{1'}$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform sind $X^2$ und $X^{2'}$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform ist $X^2$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform ist $X^{2'}$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform sind $X^3$ und $X^{3'}$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform ist $X^3$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform ist $X^{3'}$ ausgewählt aus: *tert*-Butyloxycarbonyl, Methyloxycarbonyl, Benzyloxycarbonyl, Phenyloxycarbonyl, Acetyl, Trifluoracetyl, Benzoyl.

In einer nicht erfindungsgemäßen Ausführungsform sind $X^1$ und $X^{1'}$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform ist $X^1$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform ist $X^{1'}$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform sind $X^2$ und $X^{2'}$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform ist $X^2$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform ist $X^{2'}$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform sind $X^3$ und $X^{3'}$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform ist $X^3$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform ist $X^{3'}$ ausgewählt aus: *tert*-Butyloxycarbonyl, Methyloxycarbonyl, Phenyloxycarbonyl, Acetyl, Trifluoracetyl, Benzoyl.

In einer nicht erfindungsgemäßen Ausführungsform sind $X^1$ und $X^{1'}$ ausgewählt aus: *tert*-Butyloxycarbonyl, Methyloxycarbonyl, Phenyloxycarbonyl, Trifluoracetyl, Benzoyl.

In einer nicht erfindungsgemäßen Ausführungsform ist $X^1$ ausgewählt aus: *tert*-Butyloxycarbonyl, Methyloxycarbonyl, Phenyloxycarbonyl, Trifluoracetyl, Benzoyl.

In einer nicht erfindungsgemäßen Ausführungsform ist $X^{1'}$ ausgewählt aus: *tert*-Butyloxycarbonyl, Methyloxycarbonyl, Phenyloxycarbonyl, Trifluoracetyl, Benzoyl.

In einer nicht erfindungsgemäßen Ausführungsform sind $X^2$ und $X^{2'}$ ausgewählt aus: *tert*-Butyloxycarbonyl, Methyloxycarbonyl, Phenyloxycarbonyl, Trifluoracetyl, Benzoyl.

In einer nicht erfindungsgemäßen Ausführungsform ist $X^2$ ausgewählt aus: *tert*-Butyloxycarbonyl, Methyloxycarbonyl, Phenyloxycarbonyl, Trifluoracetyl, Benzoyl.

In einer nicht erfindungsgemäßen Ausführungsform ist $X^{2'}$ ausgewählt aus: *tert*-Butyloxycarbonyl, Methyloxycarbonyl, Phenyloxycarbonyl, Trifluoracetyl, Benzoyl.

In einer nicht erfindungsgemäßen Ausführungsform sind $X^3$ und $X^{3'}$ ausgewählt aus: *tert*-Butyloxycarbonyl, Methyloxycarbonyl, Phenyloxycarbonyl, Trifluoracetyl, Benzoyl.

In einer nicht erfindungsgemäßen Ausführungsform ist $X^3$ ausgewählt aus: *tert*-Butyloxycarbonyl, Methyloxycarbonyl, Phenyloxycarbonyl, Trifluoracetyl, Benzoyl.

In einer nicht erfindungsgemäßen Ausführungsform ist $X^{3'}$ ausgewählt aus: *tert*-Butyloxycarbonyl, Methyloxycarbonyl, Phenyloxycarbonyl, Trifluoracetyl, Benzoyl.

In einer bevorzugten nicht erfindungsgemäßen Ausführungsform handelt es sich bei den Resten $X^1$ und $X^{1'}$, $X^2$ und $X^{2'}$, $X^3$ und $X^{3'}$ um Schutzgruppen. Also solche chemischen Gruppen, welche wieder abgespaltet werden können. Dadurch, dass $X^1$ und $X^{1'}$, $X^2$ und $X^{2'}$, $X^3$ und $X^{3'}$ jeweils paarweise für unterschiedliche Reste stehen, können die unterschiedlichen Schutzgruppen selektiv abgespalten werden (orthogonal Schutzgruppen).

Orthogonalität von Schutzgruppen bedeutet, dass sich bei Verwendung mehrerer Schutzgruppen verschiedenen Typs jede Schutzgruppe einzeln und in einer beliebigen Reihenfolge aufgrund der verschiedenen Abspaltreagenzien abspalten lässt, ohne dass eine der anderen Schutzgruppen angegriffen wird.

So kann beispielsweise $X^1$ abgespalten werden und $X^{1'}$ verbleibt am Molekül, oder kann in einem weiteren Reaktionsschritt abgespalten werden. Ebenso ist es umgekehrt denkbar, dass $X^{1'}$ selektiv abgespalten wird und $X^1$ am Molekül verbleibt.

Entsprechendes gilt analog für die Paarungen $X^2$ und $X^{2'}$, $X^3$ und $X^{3'}$.

In einer nicht erfindungsgemäßen Ausführungsform sind $R^1$, $R^2$, $R^3$, $R^4$, $R^{1'}$,$R^{2'}$, $R^{3'}$, $R^{4'}$ ausgewählt aus: -H, -($C_1$-$C_{12}$)-Alkyl, -O-($C_1$-$C_{12}$)-Alkyl, -O-($C_6$-$C_{20}$)-Aryl, -S-Alkyl, -S-Aryl, Halogen.

In einer nicht erfindungsgemäßen Ausführungsform sind $R^1$, $R^2$, $R^3$, $R^4$, $R^{1'}$, $R^{2'}$, $R^{3'}$, $R^{4'}$ ausgewählt aus: -H, -($C_1$-$C_{12}$)-Alkyl, -O-($C_1$-$C_{12}$)-Alkyl, -O-($C_6$-$C_{20}$)-Aryl.

In einer nicht erfindungsgemäßen Ausführungsform sind $R^{5'}$, $R^{6'}$, $R^{7'}$, $R^{8'}$, $R^{9'}$, $R^{10'}$ ausgewählt aus: -H, -($C_1$-$C_{12}$)-Alkyl, -O-($C_1$-$C_{12}$)-Alkyl, -O-($C_6$-$C_{20}$)-Aryl, -S-Alkyl, -S-Aryl, Halogen.

In einer nicht erfindungsgemäßen Ausführungsform sind $R^{5'}$, $R^{6'}$, $R^{7'}$, $R^{8'}$, $R^{9'}$, $R^{10'}$ ausgewählt aus: -H, -($C_1$-$C_{12}$)-Alkyl, -O-($C_1$-$C_{12}$)-Alkyl, -O-($C_6$-$C_{20}$)-Aryl.

In einer nicht erfindungsgemäßen Ausführungsform sind $R^5$, $R^6$, $R^7$, $R^8$, $R^9$, $R^{10}$ ausgewählt sind aus: -H, -($C_1$-$C_{12}$)-Alkyl, -O-($C_1$-$C_{12}$)-Alkyl, -O-($C_6$-$C_{20}$)-Aryl, -S-Alkyl, -S-Aryl, Halogen.

In einer Ausführungsform sind $R^5$, $R^6$, $R^7$, $R^8$, $R^9$, $R^{10}$ ausgewählt sind aus: -H, -($C_1$-$C_{12}$)-Alkyl, -O-($C_1$-$C_{12}$)-Alkyl, -O-($C_6$-$C_{20}$)-Aryl.

In einer nicht erfindungsgemäßen Ausführungsform weist die Verbindung eine der beiden allgemeinen Strukturen (**I**) oder (**II**) auf.

In einer nicht erfindungsgemäßen Ausführungsform weist die Verbindung die allgemeine Struktur (**I**) auf.

In einer nicht erfindungsgemäßen Ausführungsform weist die Verbindung die allgemeine Struktur (**II**) auf.

In einer nicht erfindungsgemäßen Ausführungsform weist die Verbindung die allgemeine Struktur (**III**) auf.

Beansprucht wird ein Verfahren zu deren Herstellung von 2,2'-Diaminobiarylen

Verfahren zur Herstellung von 2,2'-Diaminobiaryle umfassend die Verfahrensschritte:

a) Umsetzung einer Verbindung gemäß der Formel (**IVa**) oder (**Va**):

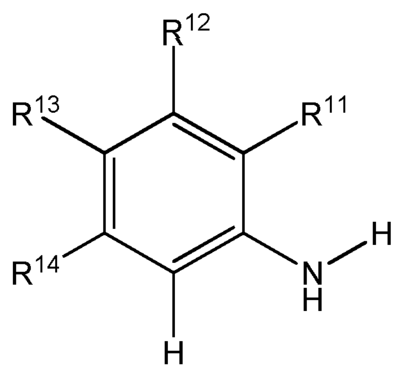

(**IVa**)

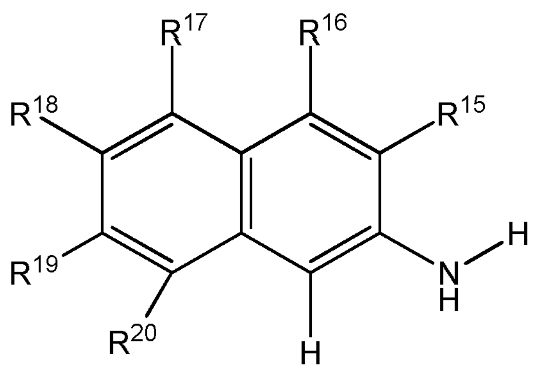

(**Va**)

wobei (**IVa**) mit zu (**IVb1**) beziehungsweise (**IVb2**), oder (**Va**) zu (**Vb**) umgesetzt wird:

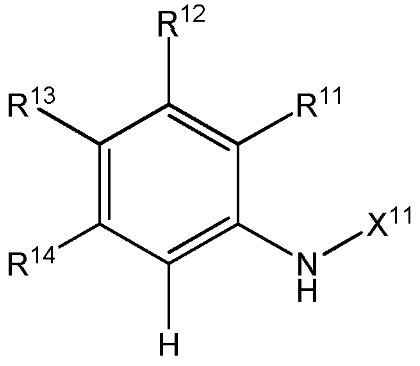

(**IVb1**)

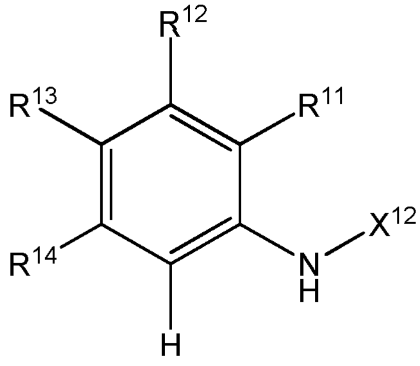

(**IVb2**)

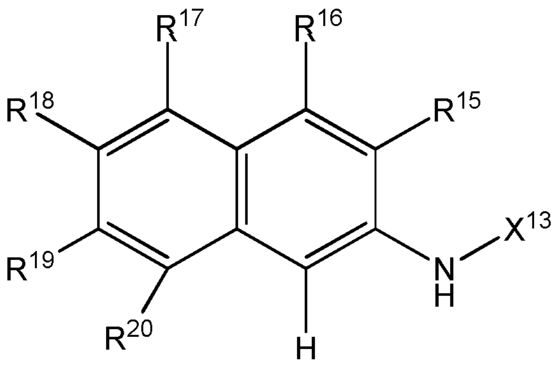

(**Vb**)

b) Umsetzung einer Verbindung gemäß der Formel (**VIa**) oder (**VIIa**):

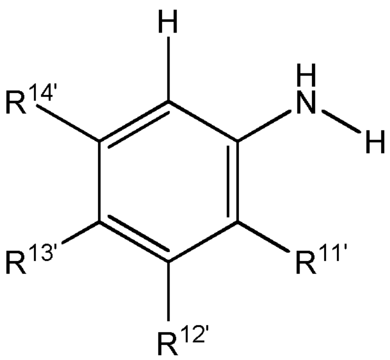

(**VIa**)

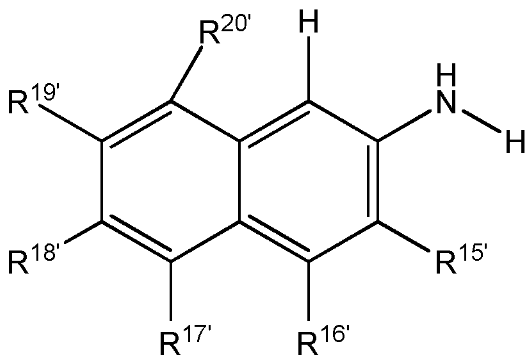

(**VIIa**)

wobei (**VIa**) zu (**VIb**), oder (**VIIa**) zu (**VIIb1**) beziehungsweise (**VIIb2**) umgesetzt wird:

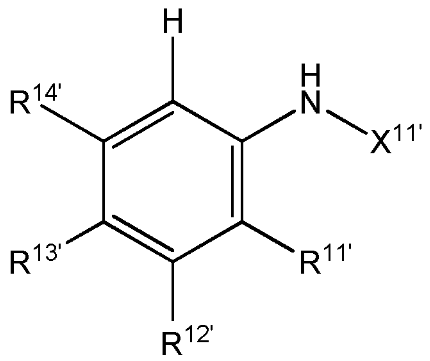

(**VIb**)

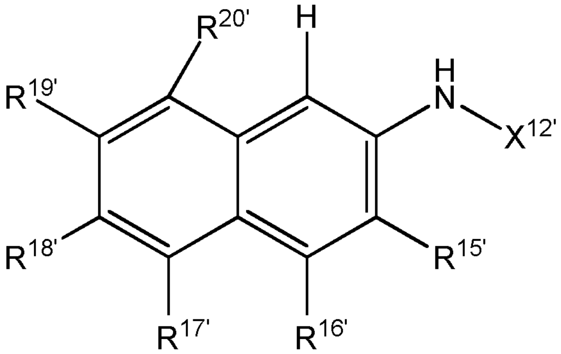

(**VIIb1**)

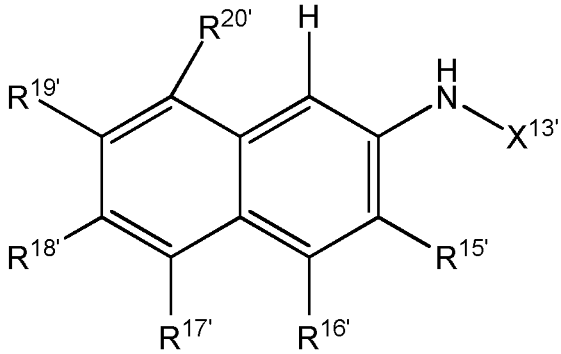

(**VIIb2**)

c) elektrochemische Kupplung von:

- (**IVb1**) mit (**VIb**) zu (**I***) oder,
- (**IVb2**) mit (**VIIb1**) zu (**II***) oder,
- (**Vb**) mit (**VIIb2**) zu (**III***),

wobei jeweils die Verbindung mit dem höheren Oxidationspotential im Überschuss eingesetzt wird:

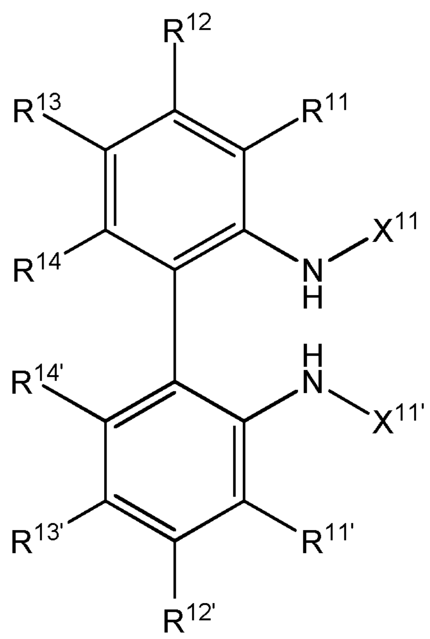

(**I***)

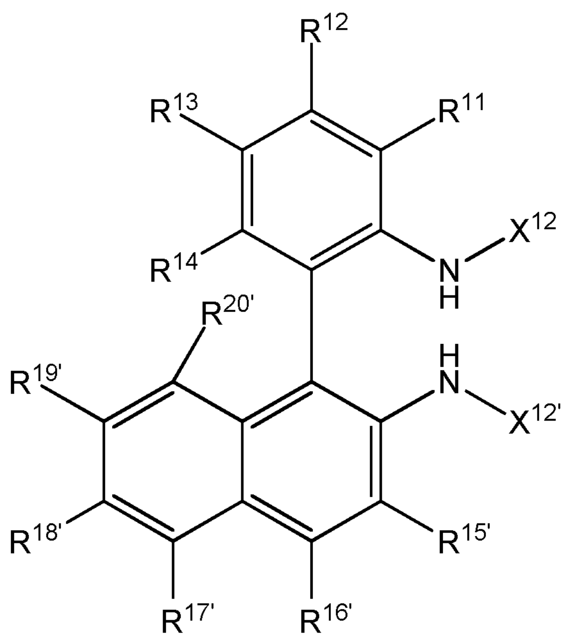

(**II***)

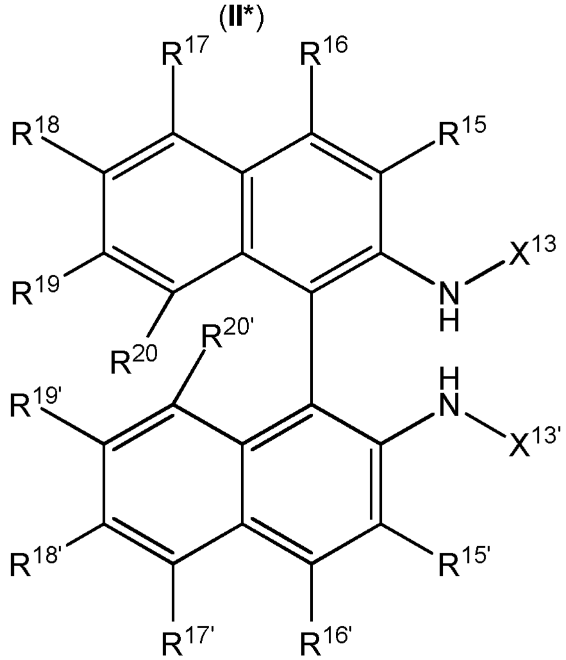

(**III***)

wobei

$R^{11}$, $R^{12}$, $R^{13}$, $R^{14}$, $R^{11'}$, $R^{12'}$, $R^{13'}$, $R^{14'}$ ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -S-Alkyl, -S-Aryl, Halogen, -COO-($C_1$-$C_{12}$)-Alkyl, -CONH-($C_1$-$C_{12}$)-Alkyl, -CO-($C_1$-$C_{12}$)-Alkyl, -CO-($C_6$-$C_{20}$)-Aryl, -COOH, -OH, -$SO_3H$, -CN, -N[($C_1$-$C_{12}$)-Alkyl]$_2$;
$R^{15}$, $R^{16}$, $R^{17}$, $R^{18}$, $R^{19}$, $R^{20}$, $R^{15'}$, $R^{16'}$, $R^{17'}$, $R^{18'}$, $R^{19'}$, $R^{20'}$ ausgewählt sind aus:
-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -S-Alkyl, -S-Aryl, Halogen, -COO-($C_1$-$C_{12}$)-Alkyl, -CONH-($C_1$-$C_{12}$)-Alkyl, -CO-($C_1$-$C_{12}$)-Alkyl, -CO-($C_6$-$C_{20}$)-Aryl, -COOH, -OH, -$SO_3H$, -N[($C_1$-$C_{12}$)-Alkyl]$_2$;
wobei die genannten Alkyl- und Arylgruppen substituiert sein können;
$X^{11}$ und $X^{11'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^{11}$ nicht für den gleichen Rest steht wie $X^{11'}$;

$X^{12}$ und $X^{12'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^{12}$ nicht für den gleichen Rest steht wie $X^{12'}$;

$X^{13}$ und $X^{13'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^{13}$ nicht für den gleichen Rest steht wie X13'.

Die in Zusammenhang mit M. Shi, W.-L. Duan, G.-B. Rong, Chirality 2004, 16, 642-651, diskutierten Nachteile weist das erfindungsgemäße Verfahren nicht auf, da die Aminoaryle zunächst selektiv geschützt und erst im Anschluss elektrochemisch gekuppelt werden. Dadurch sind völlig neue Schutzgruppenkombinationen in den 2,2'-Diaminobiaryle möglich, die sich auf dem in Stand der Technik beschriebenen Wege nicht herstellen lassen. Ferner vereinfacht sich

die Aufarbeitung stark, da die Einführung der Schutzgruppe viel selektiver möglich ist. Im Fall der Schützung eines 2,2'-Diaminobiaryls so wie es im Stand der Technik beschrieben wird, existieren zwei reaktiven Gruppen im Molekül, also zwei Aminofunktionen, die auch unter bestimmten Bedingungen zumindest in Spuren beide reagieren könnten. Dadurch wird die anschließende Aufarbeitung deutlich erschwert und es sind unter Umständen mehrere Aufreinigungsschritte notwendig, wodurch die Ausbeuten geringer werden und deutlich mehr Abfälle (Lösungsmittel) produziert werden. Weiterhin werden hier anders als im Stand der Technik 2,2'-Diaminobiaryle mit Gruppen, die eine C=O oder S-Gruppe enthalten, beansprucht, wobei die Gruppen an den beiden Stickstoffatomen verschieden sind.

In einer Variante des Verfahrens sind $X^{11}$ und $X^{11'}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens ist $X^{11}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens ist $X^{11'}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens sind $X^{12}$ und $X^{12'}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens ist $X^{12}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens ist $X^{12'}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens sind $X^{12}$ und $X^{13'}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens ist $X^{13}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens ist $X^{13'}$ ausgewählt aus:
*tert*-Butyloxycarbonyl, Methyloxycarbonyl, Benzyloxycarbonyl, Phenyloxycarbonyl, Acetyl, Trifluoracetyl, Benzoyl.

In einer Variante des Verfahrens sind $X^{11}$ und $X^{11'}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens ist $X^{11}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens ist $X^{11'}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens sind $X^{12}$ und $X^{12'}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens ist $X^{12}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens ist $X^{12'}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens sind $X^{12}$ und $X^{13'}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens ist $X^{13}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens ist $X^{13'}$ ausgewählt aus:
*tert*-Butyloxycarbonyl, Methyloxycarbonyl, Phenyloxycarbonyl, Acetyl, Trifluoracetyl, Benzoyl.

In einer Variante des Verfahrens sind $X^{11}$ und $X^{11'}$ ausgewählt aus:
*tert*-Butyloxycarbonyl, Methyloxycarbonyl, Phenyloxycarbonyl, Trifluoracetyl, Benzoyl.

In einer Variante des Verfahrens ist $X^{11}$ ausgewählt aus:
*tert*-Butyloxycarbonyl, Methyloxycarbonyl, Phenyloxycarbonyl, Trifluoracetyl, Benzoyl.

In einer Variante des Verfahrens ist $X^{11'}$ ausgewählt aus:
*tert*-Butyloxycarbonyl, Methyloxycarbonyl, Phenyloxycarbonyl, Trifluoracetyl, Benzoyl.

In einer Variante des Verfahrens sind $X^{12}$ und $X^{12'}$ ausgewählt aus:
*tert*-Butyloxycarbonyl, Methyloxycarbonyl, Phenyloxycarbonyl, Trifluoracetyl, Benzoyl.

In einer Variante des Verfahrens ist $X^{12}$ ausgewählt aus:
*tert*-Butyloxycarbonyl, Methyloxycarbonyl, Phenyloxycarbonyl, Trifluoracetyl, Benzoyl.

In einer Variante des Verfahrens ist $X^{12'}$ ausgewählt aus:
*tert*-Butyloxycarbonyl, Methyloxycarbonyl, Phenyloxycarbonyl, Trifluoracetyl, Benzoyl.

In einer Variante des Verfahrens sind $X^{12}$ und $X^{13'}$ ausgewählt aus:
*tert*-Butyloxycarbonyl, Methyloxycarbonyl, Phenyloxycarbonyl, Trifluoracetyl, Benzoyl.

In einer Variante des Verfahrens ist $X^{13}$ ausgewählt aus:

*tert*-Butyloxycarbonyl, Methyloxycarbonyl, Phenyloxycarbonyl, Trifluoracetyl, Benzoyl.

In einer Variante des Verfahrens ist $X^{13'}$ ausgewählt aus:
*tert*-Butyloxycarbonyl, Methyloxycarbonyl, Phenyloxycarbonyl, Trifluoracetyl, Benzoyl.

In einer nicht erfindungsgemäßen Variante des Verfahrens handelt es sich bei den Resten $X^{11}$ und $X^{11'}$, $X^{12}$ und $X^{12'}$, $X^{13}$ und $X^{13'}$ um Schutzgruppen. Also solche chemischen Gruppen, welche unter den jeweils geeigneten Bedingungen wieder abgespaltet werden können. Dadurch, dass $X^{11}$ und $X^{11'}$, $X^{12}$ und $X^{12'}$, $X^{13}$ und $X^{13'}$ jeweils paarweise für unterschiedliche Reste stehen, können die unterschiedlichen Schutzgruppen selektiv abgespalten werden (orthogonal Schutzgruppen). Orthogonalität von Schutzgruppen bedeutet, dass sich bei Verwendung mehrerer Schutzgruppen verschiedenen Typs jede Schutzgruppe einzeln und in einer beliebigen Reihenfolge aufgrund der verschiedenen Abspaltreagenzien abspalten lässt, ohne dass eine der anderen Schutzgruppen angegriffen wird.

So kann beispielsweise $X^{11}$ abgespalten werden und $X^{11'}$ verbleibt am Molekül, oder kann in einem weiteren Reaktionsschritt abgespalten werden. Ebenso ist es umgekehrt denkbar, dass $X^{11'}$ selektiv abgespalten wird und $X^{11}$ am Molekül verbleibt.

Entsprechendes gilt analog für die Paarungen $X^{12}$ und $X^{12'}$, $X^{13}$ und $X^{13'}$.

In einer Variante des Verfahrens sind $R^{11}$, $R^{12}$, $R^{13}$, $R^{14}$, $R^{11'}$, $R^{12'}$, $R^{13'}$, $R^{14'}$ ausgewählt aus: -H, -($C_1$-$C_{12}$)-Alkyl, -O-($C_1$-$C_{12}$)-Alkyl, -O-($C_6$-$C_{20}$)-Aryl, -S-Alkyl, -S-Aryl, Halogen.

In Variante des Verfahrens sind $R^{11}$, $R^{12}$, $R^{13}$, $R^{14}$, $R^{11'}$, $R^{12'}$, $R^{13'}$, $R^{14'}$ ausgewählt aus: -H, -($C_1$-$C_{12}$)-Alkyl, -O-($C_1$-$C_{12}$)-Alkyl, -O-($C_6$-$C_{20}$)-Aryl.

In einer Variante des Verfahrens sind $R^{15'}$, $R^{16'}$, $R^{17'}$, $R^{18'}$, $R^{19'}$, $R^{20'}$ ausgewählt aus: -H, -($C_1$-$C_{12}$)-Alkyl, -O-($C_1$-$C_{12}$)-Alkyl, -O-($C_6$-$C_{20}$)-Aryl, -S-Alkyl, -S-Aryl, Halogen.

In einer Variante des Verfahrens sind $R^{15'}$, $R^{16'}$, $R^{17'}$, $R^{18'}$, $R^{19'}$, $R^{20'}$ ausgewählt aus: -H, -($C_1$-$C_{12}$)-Alkyl, -O-($C_1$-$C_{12}$)-Alkyl, -O-($C_6$-$C_{20}$)-Aryl.

In einer Variante des Verfahrens sind $R^{15}$, $R^{16}$, $R^{17}$, $R^{18}$, $R^{19}$, $R^{20}$ ausgewählt sind aus: -H, -($C_1$-$C_{12}$)-Alkyl, -O-($C_1$-$C_{12}$)-Alkyl, -O-($C_6$-$C_{20}$)-Aryl, -S-Alkyl, -S-Aryl, Halogen.

In einer Variante des Verfahrens sind $R^{15}$, $R^{16}$, $R^{17}$, $R^{18}$, $R^{19}$, $R^{20}$ ausgewählt sind aus: -H, -($C_1$-$C_{12}$)-Alkyl, -O-($C_1$-$C_{12}$)-Alkyl, -O-($C_6$-$C_{20}$)-Aryl.

In einer Variante des Verfahrens umfasst dieses den zusätzlichen Verfahrensschritt:
d1) selektive Abspaltung von $X^{11}$.

In einer Variante des Verfahrens umfasst dieses den zusätzlichen Verfahrensschritt:
d2) selektive Abspaltung von $X^{11'}$.

In einer Variante des Verfahrens umfasst dieses den zusätzlichen Verfahrensschritt:
d3) selektive Abspaltung von $X^{12}$.

In einer Variante des Verfahrens umfasst dieses den zusätzlichen Verfahrensschritt:
d4) selektive Abspaltung von $X^{12'}$.

In einer Variante des Verfahrens umfasst dieses den zusätzlichen Verfahrensschritt:
d5) selektive Abspaltung von $X^{13}$.

In einer Variante des Verfahrens umfasst dieses den zusätzlichen Verfahrensschritt:
d6) selektive Abspaltung von $X^{13'}$.

Durch elektrochemische Kupplung (Verfahrensschritt c)) werden Biaryldiamine hergestellt, ohne das organische Oxidationsmittel zugegeben, unter Feuchtigkeitsausschluss gearbeitet oder anaerobe Reaktionsführungen eingehalten werden müssen. Durch diese direkte Methode der C,C-Kupplung wird eine kostengünstige und umweltschonende Alternative zu bisher bestehenden mehrstufigen klassisch organischen Syntheserouten eröffnet.

Der Verfahrensschritt c) kann an unterschiedlichen Kohlenstoff- (Glaskohlenstoff, Bor-dotierter Diamant, Graphiten, Kohlenstoffasern, Nanotubes, u.a.), Metalloxid- und Metallelektroden durchgeführt werden. Dabei werden Stromdichten im Bereich von 1-50 mA/$cm^2$ appliziert.

Die elektrochemische Kupplung (Verfahrensschritt c)) wird in den üblichen, bekannten Elektrolysezellen durchgeführt.

In einer Variante des Verfahrens wird das zweite Aminoaryl gegenüber dem ersten Aminoaryl mindestens in der doppelten Menge eingesetzt.

In einer Variante des Verfahrens liegt das Verhältnis von erstem Aminoaryl zum zweiten Aminoaryl im Bereich von 1:2 bis 1:4.

Wenn erforderlich kann der Reaktion ein Leitsalz zugegeben werden.

In einer Variante des Verfahrens ist das Leitsalz ausgewählt aus der Gruppe von Alkali-, Erdalkali-, Tetra($C_1$-$C_6$-alkyl) -ammonium-,1,3-Di($C_1$-$C_6$-alkyl)imidazolium oder Tetra($C_1$-$C_6$-alkyl)-phosphoniumsalzen.

In einer Variante des Verfahrens sind die Gegenionen der Leitsalze ausgewählt aus der Gruppe von Sulfat, Hydrogensulfat, Alkylsulfate, Arylsulfate, Alkylsulfonate, Arylsulfonate, Halogenide, Phosphate, Carbonate, Alkylphosphate, Alkylcarbonate, Nitrat, Tetrafluorborat, Hexafluorphosphat, Hexafluorsilikat, Fluorid und Perchlorat.

In einer Variante des Verfahrens ist das Leitsalz ausgewählt aus Tetra-($C_1$-$C_6$-alkly)ammoniumsalzen und das

Gegenion ausgewählt aus Sulfat, Alkylsulfat, Arylsulfat.

Die Aufarbeitung und Gewinnung der Biaryldiamine ist sehr einfach und erfolgt nach Beendigung der Reaktion nach allgemein gängigen Trennmethoden. Zunächst wird die Elektrolytlösung erst einmal destilliert und die einzelnen Verbindungen in Form von unterschiedlichen Fraktionen getrennt gewonnen. Eine weitere Reinigung kann beispielsweise durch Kristallisation, Destillation, Sublimation oder chromatographisch erfolgen.

Ein Problem, das bei der elektrochemischen Kupplung von unterschiedlichen Molekülen auftritt ist, dass die Reaktionspartner in der Regel unterschiedliche Oxidationspotentiale $E_{Ox}$ haben. Dies hat zur Folge, dass das Molekül mit dem niedrigeren Oxidationspotential ein höheres Bestreben hat ein Elektron ($e^-$) an die Anode und ein $H^+$-Ion an z.B. das Lösungsmittel abzugeben, als das Molekül mit dem niedrigeren Oxidationspotential. Berechnen lässt sich das Oxidationspotential $E_{Ox}$ über die Nernstsche-Gleichung:

$$E_{Ox} = E^{\circ} + (0{,}059/n) * \lg([Ox]/[Red])$$

$E_{Ox}$: Elektrodenpotential für die Oxidationsreaktion (= Oxidationspotential)
$E^{\circ}$: Standardelektrodenpotential
*n*: Anzahl der übertragenen Elektronen
*[Ox]*: Konzentration der oxidierten Form
*[Red]:* Konzentration der reduzierten Form

Würde man ein Verfahren, welches von der Kupplung zwei identischer Aniline bekannt ist, auf zwei unterschiedliche Aniline anwenden, so hätte dies zur Folge, dass überwiegend Radikale des Moleküls entstehen würden, welches ein niedrigeres Oxidationspotential hat, und diese würde dann mit sich selbst reagieren. Als deutlich überwiegendes Hauptprodukt würde man also ein Biaryldiamin erhalten, welches aus zwei gleichen Anilinen entstanden ist.

Die Oxidationspotentiale der jeweiligen Anilin- und/oder Naphthylaminderivate sind sowohl von der jeweils verwendeten Schutzgruppe, als auch von der Struktur des Substrats selbst abhängig. Je nach verwendeter Schutzgruppe ist eine Änderung des Oxidationspotentials um mehrere hundert Millivolt möglich. Diese Einstellung der Oxidationspotentiale ist durch elektronenziehende oder elektronenschiebende Gruppen, aber auch durch unterschiedliche Größen und die damit verbundene Sterik möglich. Das erfindungsgemäße Verfahren eröffnet über die Schutzgruppen somit eine zusätzliche Möglichkeit das Oxidationspotential der Anilinbeziehungsweise Naphthylaminderivate gezielt einzustellen.

Des Weiteren ist es möglich, durch die gezielte Zugabe von protischen Additiven, wie Methanol oder Wasser zum Elektrolyten (wie HFIP: 1,1,1,3,3,3-Hexafluor-2-propanol) die Oxidationspotentiale der eingesetzter Substrate zu verschieben.

Weiterhin ist durch das Vorhandensein unterschiedlicher Schutzgruppen eine selektive Entschützung möglich (orthogonale Schutzgruppen). Die selektive Entschützung lässt sich durch die Wahl von Basen, Lewis- oder Brønstedsäuren und verwendeten Lösungsmitteln erreichen. Ist der Zugang zu einer bestimmten Aminofunktion nicht auf direktem Wege möglich, kann ein Umweg z.B. über para-Toluolsulfonsäureamiden, wie in Reaktionsschema 1 dargestellt, gewählt werden.

<u>Reaktionsschema 1:</u>

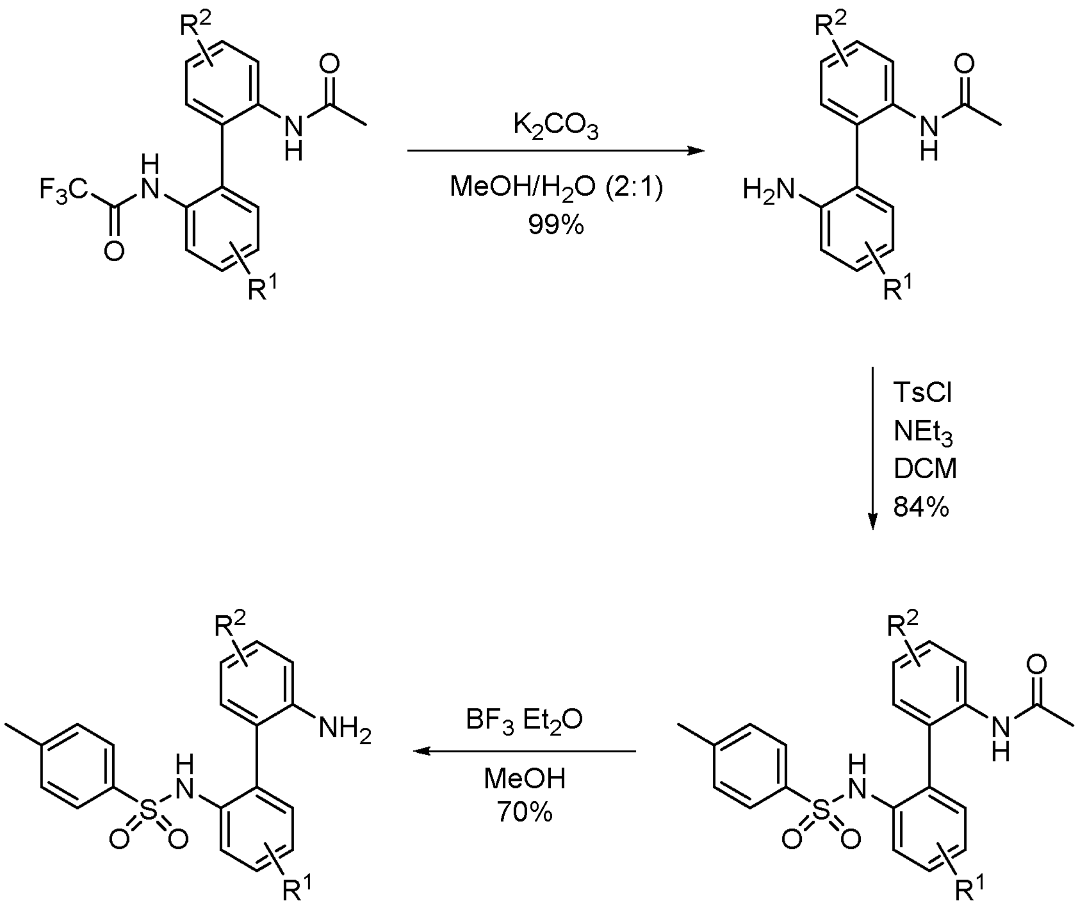


Durch elektrochemische Behandlung unterschiedlich geschützter Anilin- oder Naphthylaminderivate lassen sich unsymmetrische, mit unterschiedlichen Schutzgruppen versehene, 2,2'-Diaminobiaryle selektiv darstellen. Die gezielte Wahl der Schutzgruppen ermöglicht die Steuerung der Oxidationspotentiale. Die Erfindung ermöglicht außerdem den gezielten Zugang zu den einzelnen Aminofunktionen der 2,2'-Diaminobiaryle durch eine nachfolgende selektive Entschützung.

Reaktionsschema 2 zeigt ein allgemeines Schema der C,C-Kreuzkupplungsreaktion. Hierbei ist die Komponente **A** die Unterschusskomponente mit niedrigerem Oxidationspotential als die Überschusskomponente **B,** welche im doppelten Verhältnis eingesetzt wird. R und R' stellen die jeweiligen, gezielt wählbaren Schutzgruppen dar.

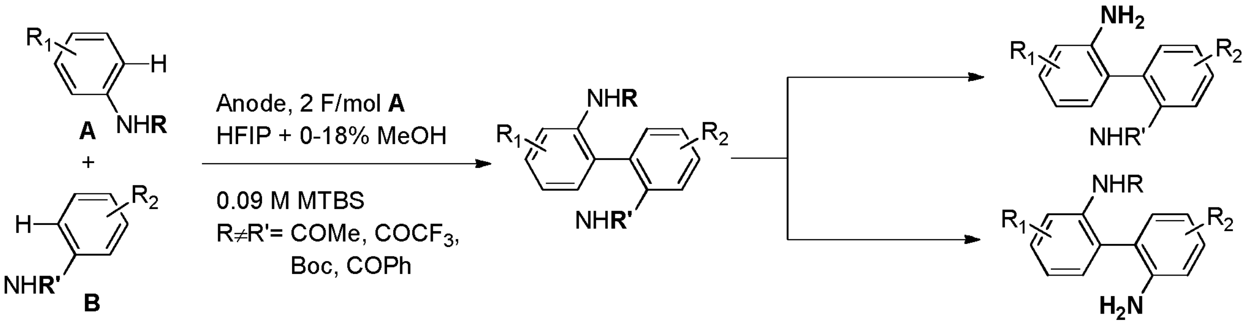


Reaktionsschema 2: Gesteuerte, elektrochemische Synthese unterschiedlich geschützter unsymmetrischer 2,2'-Diaminobiaryle und anschließend selektive Entschützung einzelner Aminofunktionen.

MTBS: $Bu_3NCH_3$ $O_3SOCH_3$
HFIP: 1,1,1,3,3,3-Hexafluor-2-propanol

Im Folgenden wird die Erfindung anhand von Ausführungsbeispielen und Figuren näher erläutert.

Die Figur 1 zeigt den schematischen Aufbau einer Reaktionsapparatur, in welcher die Kupplungsreaktion zu den entsprechenden unsymmetrischen 2,2'-Diaminobiarylen durchgeführt werden kann. Die Reaktionsapparatur umfasst Glaskohlenstoffelektroden (5), welche mit Edelstahlklemmen (4) gehalten werden. Für die Durchmischung in der Reaktionsapparatur sorgt ein Magnetrührstäbchen (6). Auf der Reaktionsapparatur sitzt ein Teflonstopfen (2), durch welchen Edelstahlhalterungen (1) für die Elektroden hindurchführen. Die Reaktionsapparatur, hier eine Becherglaszelle, verfügt über einen angesetzten Auslass (3) für einen Rückflusskühleranschluss.

Figur 2: $E_{Ox}$ in Abhängigkeit der *para*-Substituenten von Acetaniliden

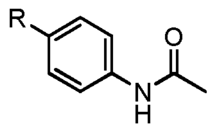

Generell ist durch Methanolzugabe ein Anstieg von $E_{ox}$ von 4-substituierten Acetaniliden zu beobachten. Hierbei fällt auf, dass $E_{ox}$ von 4-Methoxyacetanilid bei Zugabe von etwa 8% vol. MeOH sogar über $E_{ox}$ von 4-*tert*-Butylacetanilid angehoben wird. Eine Anhebung von $E_{ox}$ um bis zu 100 mV ist selektiv möglich.

Figur 3: $E_{Ox}$ in Abhängigkeit der *meta*-Substituenten von Acetaniliden

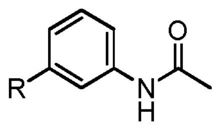

Die Methanolzugabe zu *meta*-substituierten Acetaniliden, hier am Beispiel von 3-Methoxyacetanilid gezeigt, führen zu einer nahezu linearen Erniedrigung von $E_{ox}$. Ein Abfall von 80 mV ist hier gemessen worden.

Figur 4: $E_{Ox}$ in Abhängigkeit der *ortho,para*-Disubstitution von Acetaniliden

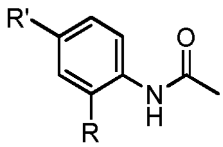

Die Änderungen von $E_{ox}$ im Falle der 2,4- Disubstitution wirken sich nur abgeschwächt auf Acetanilide aus. Lediglich ein leichter Anstieg (R= R'= Me) oder Abfall (R=Me, R'=Cl) von $E_{ox}$ ist beobachtbar. Eine Methanolzugabe bewirkt bei diesem Substitutionsmuster ein Schwanken der Oxidationspotentiale um etwa 30 mV.

Figur 5: $E_{Ox}$ in Abhängigkeit der *meta,para*-Disubstitution von Acetaniliden

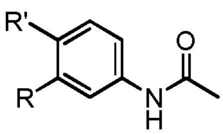

Eine starke Absenkung von $E_{ox}$ ist hingegen bei Verwendung einer 3,4-Disubstitution möglich. Hier zeigt sich, dass das Substrat mit höherer Elektronendichte (ein Benzodioxolderivat) eine deutliche Absenkung um fast 300 mV erfährt.

Figur 6: Vergleich $E_{Ox}$ in Abhängigkeit der Schützung von 3,4-Dimethoxyanilin

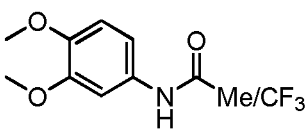

Die Verwendung von Trifluoracetyl- an Stelle von Acetylschutzgruppen bewirkt durch einen starken Elektronenzug der Fluoratome ein Anheben des $E_{ox}$ von 3,4-Dimethoxyanilin um etwa 150 mV. Gleichzeitig wird der Einfluss

von MeOH auf das Trifluoracetylderivat erheblich gesteigert. Letzteres Derivat erfährt in 1,1,1,3,3,3-Hexafluor-2-propanol (HFIP) ein Absenken von bis zu 250 mV bei MeOH-Zugabe.

Figur 7: $E_{Ox}$ in Abhängigkeit von *N*-(Naphthalin-2-yl)-acetamid

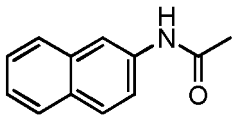

*N*-(Naphthalin-2-yl)-acetamid zeigt einen ähnlichen Verlauf wie *p*-Methoxyacetanilid. Durch Methanolzugabe kommt es zur deutlichen Anhebung von $E_{ox}$ bis etwa 15 % vol. Ein Shift von etwa 140 mV ist hierdurch möglich. Bei größeren Mengen MeOH kommt es auch hier zur Erniedrigung von $E_{ox}$.

Anhand der in den Figuren 2-7 dargestellten Ergebnisse wird deutlich, dass sich die Oxidationspotentiale durch die verschiedenen Schutzgruppen beeinflussen lassen und sich somit die elektrochemische Kupplung steuern lässt.

**Analytik**

Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma Macherey-Nagel GmbH & Co, Düren durchgeführt. Die Trennungen ohne Druckbeaufschlagung wurden an Kieselgel Geduran Si 60 (0.063-0.200 mm) der Firma Merck KGaA, Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.

Zur Dünnschichtchromatographie (DC) wurden PSC-Fertigplatten Kieselgel 60 F254 der Firma Merck KGaA, Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3g konzentrierte Schwefelsäure auf 200 mL Wasser.

Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 μm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 μm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der Ionenquelle: 200 °C) gemessen.

Schmelzpunkte

Schmelzpunkte wurden mit Hilfe des Schmelzpunktbestimmungsgerätes SG 2000 der Firma HW5, Mainz gemessen und sind unkorrigiert.

Elementaranalyse

Die Elementaranalysen wurden in der analytischen Abteilung des Institutes für Organische Chemie der Johannes Gutenberg-Universität Mainz an einem Vario EL Cube der Firma Foss-Heraeus, Haunau angefertigt.

Massenspektrometrie

Alle Elektrosprayionisation-Messungen (ESI+) wurden an einem QTof Ultima 3 der Firma Waters Micromasses, Milford, Massachusetts durchgeführt. EI-Massenspektren sowie die hochaufgelösten EI-Spektren wurden an einem

Gerät des Typs MAT 95 XL Sektorfeldgerät der Firma ThermoFinnigan, Bremen, gemessen.

NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCl3 verwendet. Die 1H- und 13C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der 1H- und 13C-Signale erfolgte teilweise mit Hilfe von H,H-COSY, H,H-NOESY, H,C-HSQC und H,C-HMBC-Spektren. Die chemischen Verschiebungen sind als $\delta$-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

Beispiele mögliche Schutzgruppen:

Carbamate:

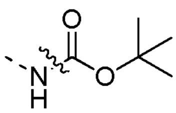
tertbutyloxycarbonyl (Boc)

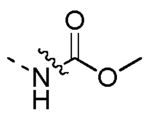
Methyloxycarbonyl

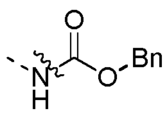
Benzyloxycarbonyl (Cbz)

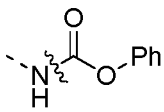
Phenyloxycarbonyl

Amide:

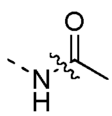
Acetyl

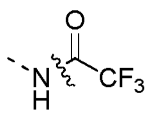
Trifluoracetyl

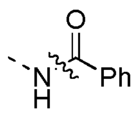
Benzoyl

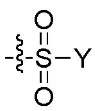
Sulfonyl

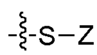
Sulfenyl

Mit Bn = Benzyl, Ph = Phenyl.
Die Reste Y und Z entsprechen der oben genannten Definition.

Das Einführen der Schutzgruppen kann beispielsweise wie in P. G. M. Wuts, T. W. Greene "Greene's Protective Groups in Organic Synthesis", fourth edition, 2007, John Wiley and Sons; Hoboken, New Jersey, beschrieben erfolgen.

**AV1: Arbeitsvorschrift zur N-Acetylierung**

Das zu schützende Anilinderivat bzw. Naphthylaminderivate (1 Äquiv.) wird in einem Rundhalskolben vorgelegt und in Dichlormethan gelöst. Unter Eiskühlung wird langsam 1.2 Äquiv. Essigsäureanhydrid zur Reaktionslösung getropft. Das Reaktionsgemisch wird nach vollständiger Zugabe bei Raumtemperatur und/oder unter Rückfluss für 24 Stunden gerührt. Nach beendeter Reaktion wird das Lösungsmittel unter vermindertem Druck entfernt und das Rohprodukt an

Kieselgel 60 als "Flashchromatographie" im Laufmittel CH:EE (4:1 bis 1:1) aufgereinigt.

**AV2: Arbeitsvorschrift zur N-2,2,2-Trifluoracetamid-Schützung**

In einem Rundhalskolben wird das zu schützende Anilinderivat bzw. Naphthylaminderivate (1 Äquiv.) in Dichlormethan gelöst vorgelegt. Zu dieser Lösung wird unter Eiskühlung und starkem rühren langsam 1.2 Äquiv. Trifluoressigsäureanhydrid zugegeben. Nach beendeter Zugabe wird der Reaktionskolben für 4-5 Stunden auf 35 °C erhitzt. Nach beendeter Reaktion wird das Lösungsmittel unter vermindertem Druck entfernt und das Rohprodukt an Kieselgel 60 als "Flashchromatographie" im Laufmittel CH:EE (4:1 bis 1:1) aufgereinigt.

**AV3: Arbeitsvorschrift zur elektrochemischen Kreuzkupplung**

In einer ungeteilten Becherglaszelle mit Glaskohlenstoffelektroden wird 3.8 mmol der Komponente **A** (vgl. Reaktionsschema 2) und 7.6 mmol der zu kuppelnden Komponente **B** (vgl. Reaktionsschema 2) in 25 mL 1,1,1,3,3,3-Hexafluorisopropanol und 0.77 g MTBS gelöst. Die Elektrolyse erfolgt galvanostatisch. Während der Elektrolyse wird die Becherglaszelle auf 50 °C mit Hilfe eines Wasserbades erhitzt und die Reaktionsmischung gerührt. Nach Ende der Elektrolyse wird der Zellinhalt in einen entsprechenden Rundhalskolben überführt und das Lösungsmittel unter vermindertem Druck am Rotationsverdampfer bei 50 °C, 200 → 90 mbar entfernt.

Elektrodenmaterial:

| | |
|---|---|
| Anode: | Glaskohlenstoff |
| Kathode: | Glaskohlenstoff |

Elektrolysebedingungen:

| | |
|---|---|
| Temperatur [T]: | 50 °C |
| Stromdichte [j]: | 2.8 mA/cm2 |
| Ladungsmenge [Q]: | 2 F (pro Unterschusskomponente) |

**AV4: Arbeitsvorschrift zur elektrochemischen Kreuzkupplung (Screening)**

In einer ungeteilten Screeningzelle werden 0.76 mmol der Komponente **A** (vgl. Reaktionsschema 2) und 1.51 mmol der zu kuppelnden Komponente **B** (vgl. Reaktionsschema 2) in 5 mL 1,1,1,3,3,3-Hexafluorisopropanol und 154 mg MTBS gelöst. Die Elektrolyse erfolgt galvanostatisch. Während der Elektrolyse wird die Screeningzelle auf 50 °C in einem Screeningblock erhitzt und die Reaktionsmischung gerührt. Nach Ende der Elektrolyse wird der Zellinhalt in einen entsprechenden Rundhalskolben überführt und das Lösungsmittel unter vermindertem Druck am Rotationsverdampfer bei 50 °C, 200 → 90 mbar entfernt.

Elektrodenmaterial:

| | |
|---|---|
| Anode: | BDD oder Glaskohlenstoff |
| Kathode: | BDD oder Glaskohlenstoff |

Elektrolysebedingungen:

| | |
|---|---|
| Temperatur [T]: | 50 °C |
| Stromdichte [j]: | 2.8 mA/cm2 |
| Ladungsmenge [Q]: | 2 F (pro Unterschusskomponente) |

**AV5: Allgemeine Arbeitsvorschrift zur Entfernung von N-2,2,2-Trifluoracetamid-Schutzgruppen**

In einem Rundhalskolben wird 1 Äquiv. des zu entschützenden Substrats, gelöst in einem Methanol Wasser Gemisch im Verhältnis 2:1, vorgelegt. Zu der Reaktionslösung werden daraufhin 10 Äquiv. Kaliumcarbonat gegeben und für vier Tage bei Raumtemperatur gerührt. Nach beendeter Reaktion entfernt man das Lösungsmittel unter vermindertem Druck. Der Rückstand wird mit Wasser aufgeschlämmt und das entschützte Produkt mit Dichlormethan extrahiert. Sofern keine quantitative Entschützung erfolgt, wird das Rohprodukt an Kieselgel 60 als "Flashchromatographie" aufgereinigt.

**AV6: Allgemeine Arbeitsvorschrift zur Entfernung von N-Acetyl-Schutzgruppen**

Das zu entschützende Substrat (1 Äquiv.) wird in einem Rundhalskolben vorgelegt und in Methanol gelöst. Unter starkem Rühren versetzt man die Reaktionslösung mit 12 Äquiv. Bortrifluoriddiethyletherat und erhitzt daraufhin das Gemisch unter Rückfluss für 18 Stunden. Die Reaktion wird durch Zugabe von 20 Äquiv. Triethylamin beendet und das Produkt fällt als Feststoff aus, welcher abfiltriert werden kann.

<u>2-(N-Acetyl)-amino-1-(2'-(N'-trifluoracetyl)-amino-4',5'-dimethoxyphenyl)-naphthalin</u>

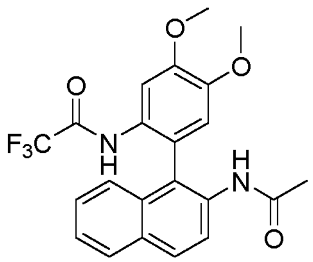

a) Synthese des 2,2'-Diaminobiaryls im Screeningmaßstab

Die Elektrolyse wird gemäß **AV4** in einer ungeteilten Screeningzelle durchgeführt. Hierfür werden 140 mg (0.76 mmol, 1.0 Äquiv.) N-(Naphthalin-2-yl)-acetamid und 377 mg (1.51 mmol, 2 Äquiv.) N-(3,4-Dimethoxyphenyl)-2,2,2-trifluoracetamid in 5 mL 1,1,1,3,3,3-Hexafluor-2-propanol (HFIP) gelöst, 154 mg MTBS zugegeben und der Elektrolyt in die Elektrolysezelle überführt. Nach der Elektrolyse werden das Lösungsmittel sowie nicht umgesetzte Edukte unter vermindertem Druck entfernt. Das Rohprodukt wird daraufhin an Kieselgel 60 als "Flashchromatographie" im Laufmittel 2:1 (CH:EE) aufgereinigt und das Produkt als farbloser Feststoff erhalten.

Mit der Screeningreaktion wurden unterschiedliche Elektrodenmaterialien untersucht. Die verwendeten Elektrodenmaterialien wurden BDD und Glaskohlenstoff gewählt, welche das C,C-Kreuzkupplungsprodukt in unterschiedlichen Ausbeuten darstellten (Tabelle 1).

Tabelle 1: Auflistung der verwendeten Eektrodenmaterialien mit den daraus resultierenden Ausbeuten.

| Elektrodenmaterial | Ausbeute |
|---|---|
| BDD | 24% (78 mg) |
| Glaskohlenstoff | 44% (144 mg) |


| Elektrodenmaterial | Ausbeute |
|---|---|
| BDD | 24% (78 mg) |
| Glaskohlenstoff | 44% (144 mg) |


b) Synthese des 2,2'-Diaminobiaryls in Becherglaszelle

Die Elektrolyse wird gemäß **AV3** in einer ungeteilten Becherglaszelle mit Glaskohlenstoffelektroden durchgeführt. Es werden 0.70 g (3.79 mmol, 1.0 Äquiv.) N-(Naphthalin-2-yl)-acetamid und 1.89 g (7.57 mmol, 2 Äquiv.) N-(3,4-Dimethoxyphenyl)-2,2,2-trifluoracetamid in 25 mL 1,1,1,3,3,3-Hexafluor-2-propanol (HFIP) gelöst, 0.77 g MTBS zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 2:1 (CH:EE) aufgereinigt und das Produkt als farbloser Feststoff erhalten.
Ausbeute: 1.02 g (62%, 2.36 mmol)
GC (Methode hart, HP-5): tR = 16.90 min

Rf (EE:CH = 2:1) = 0.5

$^1$H-NMR (300 MHz, CDCl3) δ = 2.00 (s, 3H), 3.84 (s, 3H), 4.01 (s, 3H), 6.73 (s, 1H), 7.15 (bs, 1H), 7.27 (d, J = 9 Hz, 1H), 7.44 (dt, J = 6 Hz, 7.66 (s, 1H), 7.91 (m, 3H) 7.93 (bs, 1H), 8.11 (d, 1H)

$^{13}$C-NMR (75 MHz, CDCl3) δ = 24.23, 56.33, 56.38, 107.73, 113.15, 113.63, 117.45, 120.79, 122.57, 124.55, 124.88, 125.96, 127.03, 127.54, 128.47, 130.08, 131.51, 132.36, 133.98, 148.10, 149.53, 169.47

HRMS für $C_{22}H_{19}F_3N_2O_4$ (ESI+) [M+H+]: ber.: 433.1375, gef.: 433.1375

2-(N-Acetyl)-amino-1-(2'-amino-4',5'-dimethoxyphenyl)-naphthalin

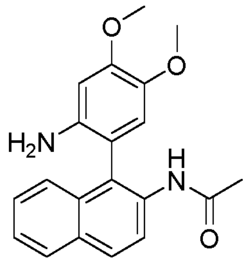

In einem Rundhalskolben wird nach **AV5** 0.65 g (1.50 mmol, 1 Äquiv.) 2-(N-Acetyl)-amino-1-(2'-(N'-trifluoracetyl)-amino-4',5'-dimethoxyphenyl)-naphthalin in 120 mL eines Methanol Wasser Gemischs im Verhältnis 2:1 gelöst. Zu dieser Lösung werden 2.07 g (15.01 mmol, 10 Äquiv.) Kaliumcarbonat hinzugegeben und das Reaktionsgemisch für vier Tage bei Raumtemperatur gerührt. Nach Ende der Reaktion wird das Lösungsmittel unter vermindertem Druck entfernt, der Rückstand mit Wasser aufgeschlämmt und mit Dirchlormethan das entschützte Produkt extrahiert.

Ausbeute: 500 mg (99%, 1.49 mmol)

GC (Methode hart, HP-5): tR = 18.68 min

Rf (EE:CH = 2:1) = 0.46

$^1$H-NMR (300 MHz, CDCl3) δ = 2.03 (s, 3H), 3.12 (bs, 2H), 3.77 (s, 3H), 3.94 (s, 3H), 6.59 (d, J = 15 Hz, 2H), 7.35-7.46 (m, 4H), 7.87 (dd, J = 9 Hz, 2H), 8.40 (d, J = 9 Hz, 1H)

$^{13}$C-NMR (75 MHz, CDCl3) δ = 24.87, 56.02, 56.58, 101.21, 111.55, 114.60, 121.36, 123.50, 125.18, 125.61, 126.81, 128.25, 129.01, 131.28, 132.77, 134.48, 137.70, 143.04, 150.28, 168.96 HRMS für $C_{20}H_{20}N_2O_3$ (ESI+) [M+H+]: ber.: 337.1552 , gef.: 337.1552

2-(N-Acetyl)-amino-1-(2'-(N'-(4-methylphenylsulfonyl))-amino-4',5'-dimethoxyphenyl)-naphthalin

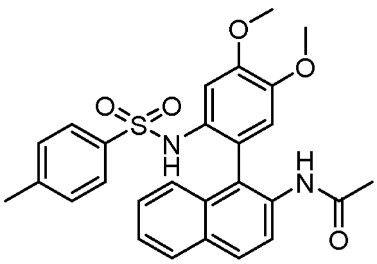

Es werden 278 mg (0.83 mmol, 1 Äquiv.) N-Acetyl-2-amino-1-(2'-amino-4',5'-dimethoxyphenyl)-naphthalin in einem Rundkolben in 110 mL Dirchlormethan vorgelegt. Zu dieser Reaktionslösung wird 173 mg (0.91 mmol, 1.1 Äquiv.) p-Methylsulfonsäurechlorid und 0.13 mL (0.91 mmol, 1.1 Äquiv.) Triethylamin gegeben und für 111 Stunden bei Raumtemperatur gerührt. Nach beendeter Reaktion wird das Lösungsmittel unter vermindertem Druck entfernt und das Rohprodukt an Kieselgel 60 als "Flashchromatographie" im Laufmittel 2:1 (CH:EE) aufgereinigt.

Ausbeute: 342 mg (84 %, 0.70 mmol)

GC (Methode hart, HP-5): tR = 16.87 min

Rf (EE:CH = 2:1) = 0.21

$^1$H-NMR (300 MHz, CDCl3) δ = 1.87 (s, 3H), 2.38 (s, 3H), 3.75 (s, 3H), 3.94 (s, 3H), 6.09 (s, 1H), 6.56 (s, 1H), 6.68 (s,

1H), 6.94 (d, J = 9 Hz, 1H), 7.10 (d, J = 6 Hz, 2H), 7.24 (t, J = 6 Hz, 1H), 7.29 (bs, 1H), 7.36 (d, J = 9 Hz, 2H), 7.42 (t, J = 6 Hz, 1H), 7.88 (dd, J = 15 Hz, J = 9 Hz, 2H), 8.32 (d, J = 9 Hz, 1H)
$^{13}$C-NMR (75 MHz, CDCl3) $\delta$ = 21.70, 24.58, 56.19, 56.27, 106.87, 113.17, 119.35, 121.46, 124.49,124.89, 125.40, 125.92, 127.40, 127.40, 127.44, 128.50, 129.74, 129.74, 129.81, 130.98, 132.28, 134.61, 136.40, 144.15, 147.38, 149.75, 168.58
HRMS für $C_{20}H_{20}N_2O_3$ (ESI+) [M+H+]: ber.: 491.1641, gef.: 491.1651

2-Amino-1-(2'-N-(4-methylphenylsulfonyl)-amino-4',5'-dimethoxyphenyl)-naphthalin

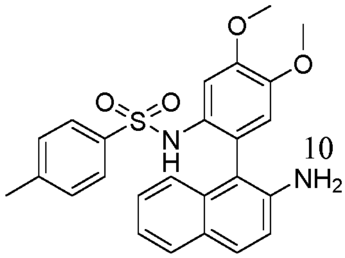

Nach **AV6** werden in 40 mL Methanol 342 mg (0.70 mmol, 1 Äquiv.) N-Acetyl-2-amino-1-(2'-N-(4-methylphenylsulfonyl)-amino-4',5'-dimethoxyphenyl)-naphthalin vorgelegt. Zu dieser Lösung gibt man unter starkem rühren 1.06 mL (8.37 mmol, 12 Äquiv.) Bortrifluoriddiethyletherat und erhitzt das Gemisch unter Rückfluss für 18 Stunden. Durch die Zugabe von 2 mL Triethylamin wird die Reaktion beendet und das Produkt fällt als Feststoff aus, welcher abfiltriert werden kann.
Ausbeute: 219 mg (70 %, 0.49 mmol)
GC (Methode hart, HP-5): tR = 15.64 min
Rf (EE:CH = 2:1) = 0.78
$^{1}$H-NMR (300 MHz, CDCl3) $\delta$ = 2.21 (s, 3H), 3.00 (bs, 2H), 3.76 (s, 3H), 3.98 (s, 3H), 6.64 (s, 1H), 6.73-6.83 (m, 4H), 7.00-7.08 (m, 2H), 7.15-7.24 (m, 3H), 7.40 (s, 1H), 7.70 (dd, J = 6 Hz, J = 9 Hz, 2H)
$^{13}$C-NMR (75 MHz, CDCl3) $\delta$ = 21.61, 56.18, 56.27, 108.18, 114.19, 114.95, 118.02, 120.61, 122.65, 123.75, 126.88, 126.98, 126.98, 128.08, 128.34, 128.49, 129.25, 129.25, 130.04, 133.51, 135.97, 140.54, 143.28, 147.20, 149.30
HRMS für $C_{20}H_{20}N_2O_3$ (ESI+) [M+H+]: ber.: 449.1535, gef.: 449.1542

2-Amino-1-(2'-N-(4-methylphenylsulfonyl)-amino-4',5'-dimethoxyphenyl)-naphthalin

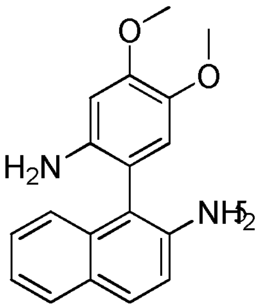

In einem Rundhalskolben werden 300 mg (0.69 mmol, 1 Äquiv.) 2-(N-Acetyl)-amino-1-(2'-(N'-trifluoracetyl)-amino-4',5'-dimethoxyphenyl)-naphthalin in 80 mL Hydrazinhydratlösung (80%ige wässrige Lösung) gelöst. Die Reaktionslösung wird unter Rückfluss für 4 Tage bei 120 °C gerührt. Nach beendeter Reaktion extrahiert man 3 mal mit je 20 mL Dichlormethan und entfernt das Lösungsmittel unter vermindertem Druck. Das Produkt wird als bräunlicher Schaum erhalten.
Ausbeute: 200 mg (98 %, 0.68 mmol)
GC (Methode hart, HP-5): tR = 17.21 min
Rf (EE:CH = 2:1) = 0.44

$^1$H-NMR (300 MHz, CDCl3) δ = 3.61 (s, 3H), 3.77 (s, 3H), 4.01 (bs, 2H), 4.77 (bs, 2H), 6.50 (s, 1H), 6.58 (s, 1H), 7.09-7.25 (m, 4H), 7.66 (d, J = 9 Hz, 1H), 7.69 (d, J = 6 Hz, 1H)
$^{13}$C-NMR (75 MHz, CDCl3) δ = 55.25, 56.37, 100.60, 111.34, 113.66, 116.07, 118.46, 120.99, 123.45, 125.97, 127.07, 127.88, 128.19, 133.70, 140.43, 140.84, 143.58, 149.32
HRMS für $C_{18}H_{18}N_2O_2$ (ESI+) [M+H+]: ber.: 295.1447, gef.: 295.1458

Die in den Beispielen gezeigten Verbindungen erfüllen die gestellte Aufgabe. Erstmalig ist es gelungen neuartige 2,2'-Diaminobiaryle in guten bis sehr guten Ausbeuten herzustellen. Hierbei wird eine völlig neuartige Synthesestrategie angewendet: Beide Aminoaryle werden zunächst unabhängig voneinander geschützt, anschließend elektrochemisch gekuppelt und können dann nach Bedarf selektiv entschützt werden. Durch diese Vorgehensweise können Verbindung mit zwei verschiedenen Schutzgruppen hergestellt werden, die aufgrund der bisherigen im Stand der Technik genannten Vorgehensweise nicht zugänglich waren.

**Patentansprüche**

**1.** Verfahren zur Herstellung von 2,2'-Diaminobiaryle umfassend die Verfahrensschritte:

a) Umsetzung einer Verbindung gemäß der Formel (**IVa**) oder (**Va**):

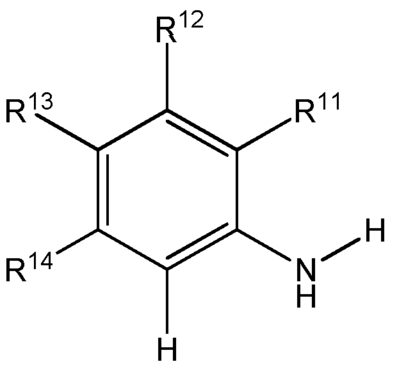

(**IVa**)

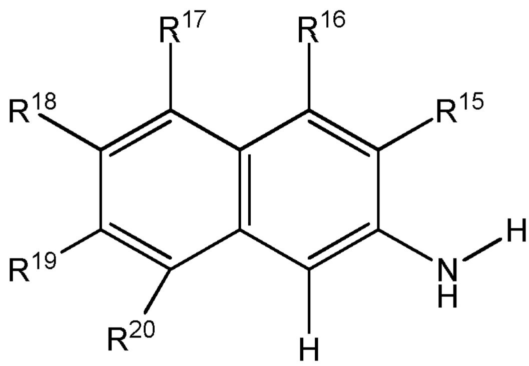

(**Va**)

wobei (**IVa**) zu **(IVb1)** beziehungsweise (**IVb2**), oder (**Va**) zu (**Vb**) umgesetzt wird:

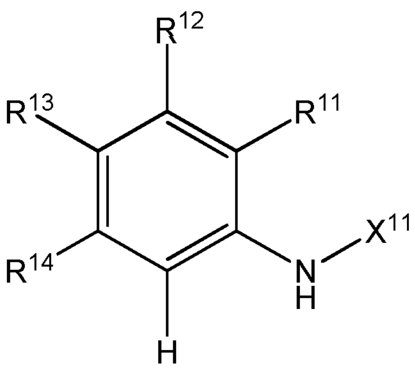

(**IVb1**)

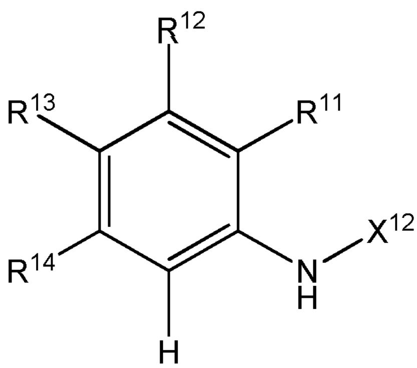

(**IVb2**)

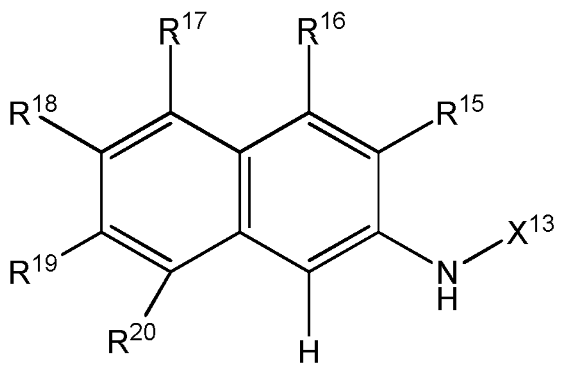

(**Vb**)

b) Umsetzung einer Verbindung gemäß der Formel (**VIa**) oder (**VIIa**):

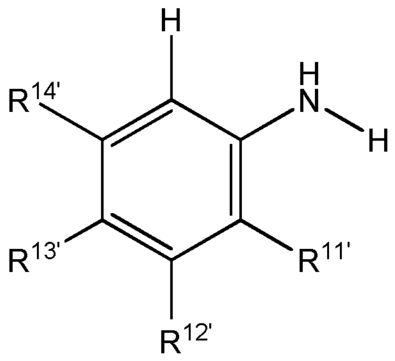

(**VIa**)

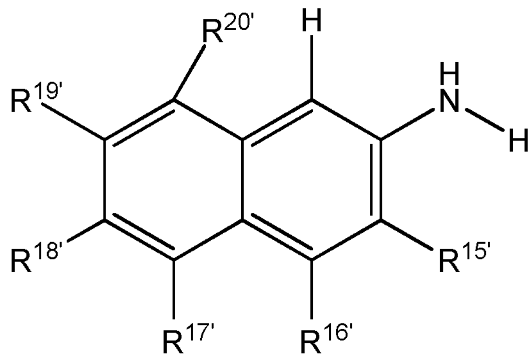

(**VIIa**)

wobei (**VIa**) zu (**VIb**), oder
(**VIIa**) zu (**VIIb1**) beziehungsweise (**VIIb2**) umgesetzt wird:

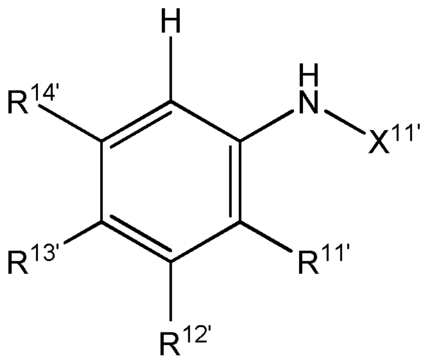

(**VIb**)

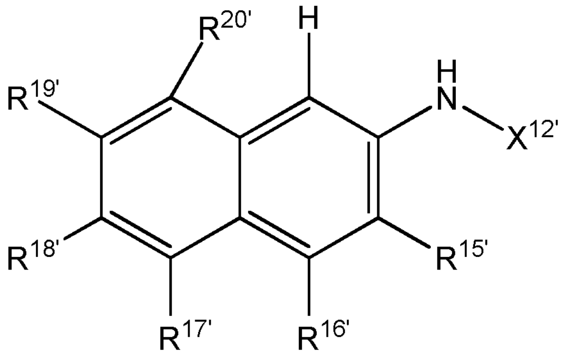

(**VIIb1**)

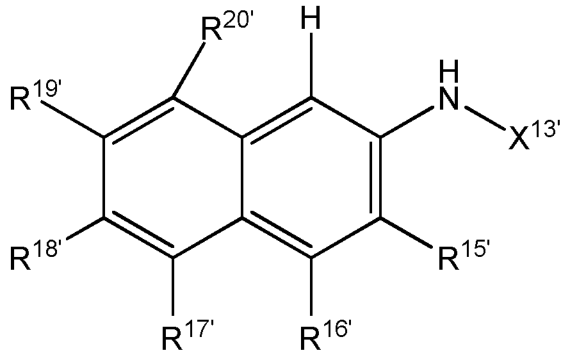

(**VIIb2**)

c) elektrochemische Kupplung von:

- **(IVb1)** mit (**VIb**) zu (**I***) oder,
- **(IVb2)** mit (**VIIb1**) zu (**II***) oder,
- **(Vb)** mit (**VIIb2**) zu (**III***),

wobei jeweils die Verbindung mit dem höheren Oxidationspotential im Überschuss eingesetzt wird:

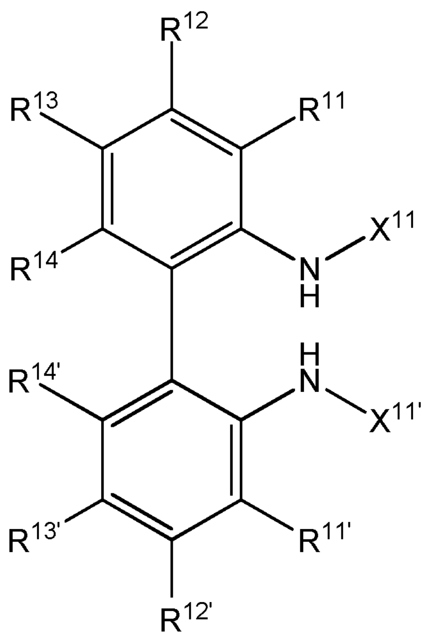

(**I***)

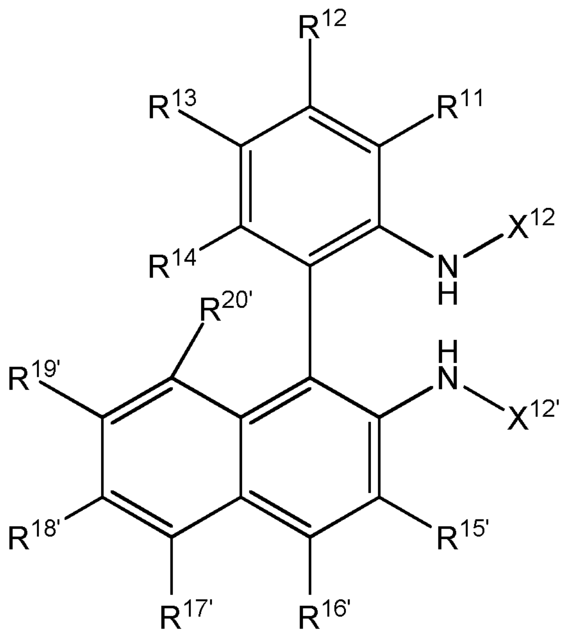

(**II***)

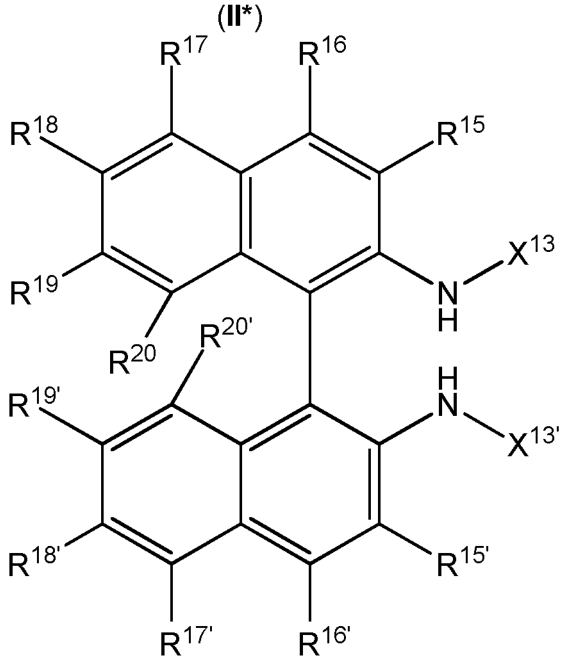

(**III***)

wobei

$R^{11}$, $R^{12}$, $R^{13}$, $R^{14}$, $R^{11'}$, $R^{12'}$, $R^{13'}$, $R^{14'}$ ausgewählt sind aus:
-H, $-(C_1-C_{12})$-Alkyl, $-O-(C_1-C_{12})$-Alkyl, $-O-(C_6-C_{20})$-Aryl, $-(C_6-C_{20})$-Aryl, -S-Alkyl, -S-Aryl, Halogen, $-COO-(C_1-C_{12})$-Alkyl, $-CONH-(C_1-C_{12})$-Alkyl, $-CO-(C_1-C_{12})$-Alkyl, $-CO-(C_6-C_{20})$-Aryl, -COOH, -OH, $-SO_3H$, -CN, $-N[(C_1-C_{12})$-Alkyl$]_2$;
$R^{15}$, $R^{16}$, $R^{17}$, $R^{18}$, $R^{19}$, $R^{20}$, $R^{15'}$, $R^{16'}$, $R^{17'}$, $R^{18'}$, $R^{19'}$, $R^{20'}$ ausgewählt sind aus:
-H, $-(C_1-C_{12})$-Alkyl, $-O-(C_1-C_{12})$-Alkyl, $-O-(C_6-C_{20})$-Aryl, $-(C_6-C_{20})$-Aryl, -S-Alkyl, -S-Aryl, Halogen, $-COO-(C_1-C_{12})$-Alkyl, $-CONH-(C_1-C_{12})$-Alkyl, $-CO-(C_1-C_{12})$-Alkyl, $-CO-(C_6-C_{20})$-Aryl, -COOH, -OH, $-SO_3H$, $-N[(C_1-C_{12})$-Alkyl$]_2$;
wobei die genannten Alkyl- und Arylgruppen substituiert sein können;

$X^{11}$ und $X^{11'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^{11}$ nicht für den gleichen Rest steht wie $X^{11'}$;

$X^{12}$ und $X^{12'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^{12}$ nicht für den gleichen Rest steht wie $X^{12'}$;

$X^{13}$ und $X^{13'}$ ausgewählt sind aus:

*tert*-Butyloxycarbonyl, Methyloxycarbonyl, Benzyloxycarbonyl, Phenyloxycarbonyl, Acetyl, Trifluoracetyl, Benzoyl, Sulfonyl, Sulfenyl,
und $X^{13}$ nicht für den gleichen Rest steht wie $X^{13'}$.

## Claims

**1.** Process for preparing 2,2'-diaminobiaryls, comprising the process steps of:

a) reacting a compound of the formula (**IVa**) or (**Va**):

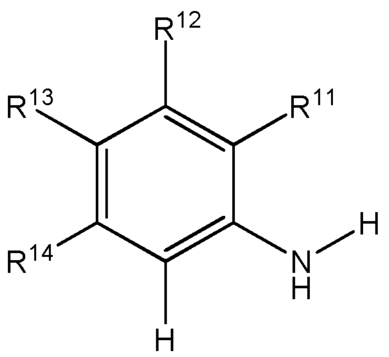

(**IVa**)

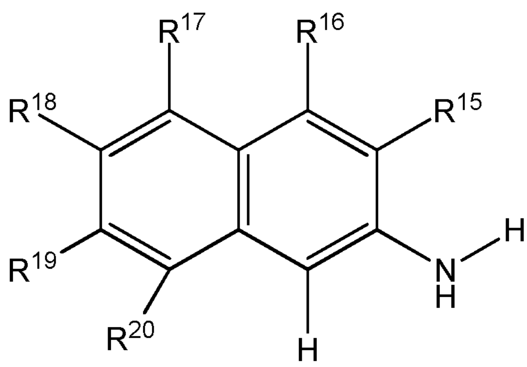

(**Va**)

with reaction of (**IVa**) to give (**IVb1**) or (**IVb2**), or of (**Va**) to give (**Vb**):

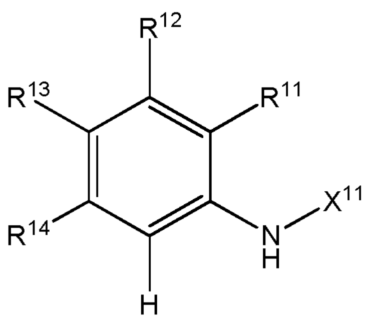

(**IVb1**)

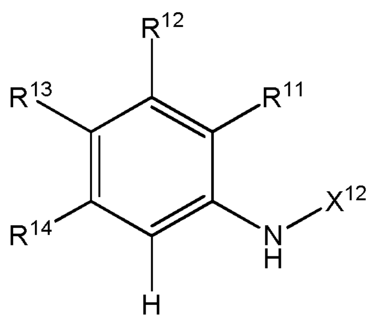

(**IVb2**)

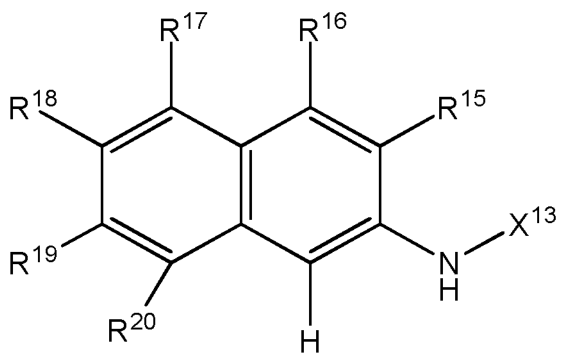

(**Vb**)

b) reacting a compound of the formula (**VIa**) or (**VIIa**) :

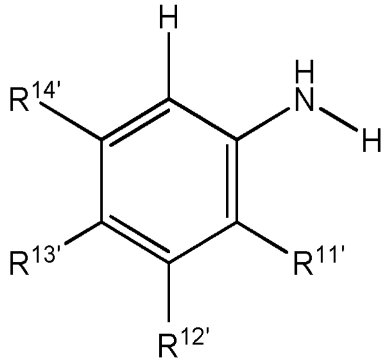

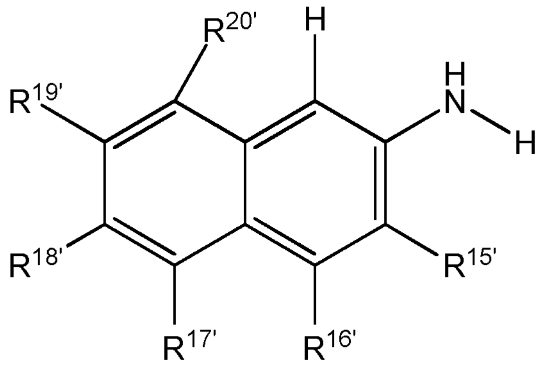

(**VIa**) (**VIIa**)

with reaction of (**VIa**) to give (**VIb**), or
of (**VIIa**) to give (**VIIb1**) or (**VIIb2**):

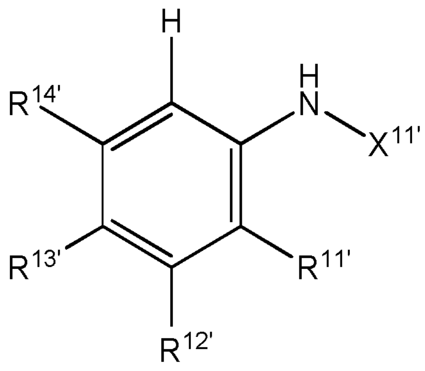

(**VIb**)

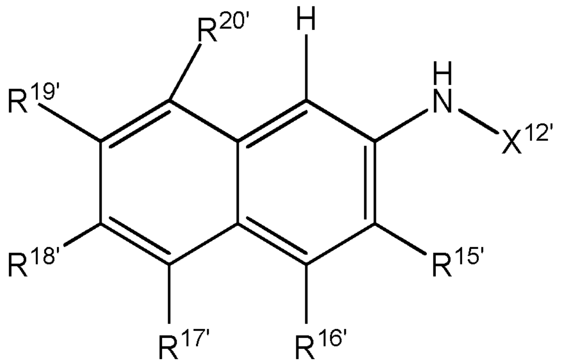

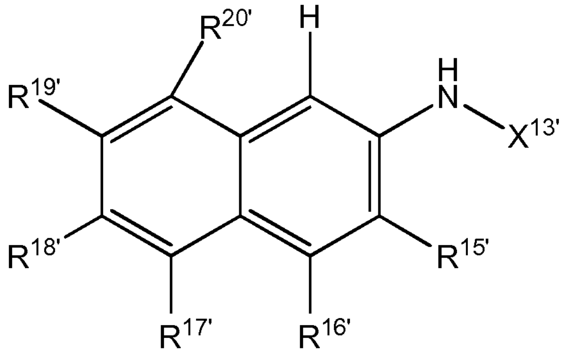

(**VIIb1**) (**VIIb2**)

c) electrochemical coupling of:

- (**IVb1**) with (**VIb**) to give (**I***), or
- (**IVb2**) with (**VIIb1**) to give (**II***), or
- (**Vb**) with (**VIIb2**) to give (**III***),

in each case with use of the compound having the higher oxidation potential in excess:

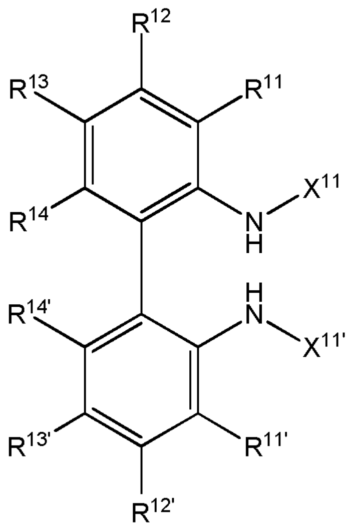

(**I***)

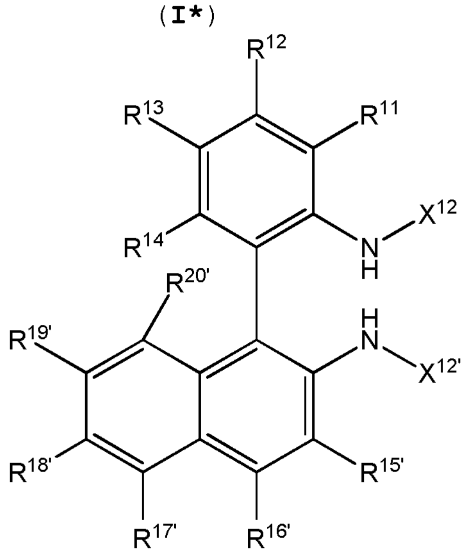

(**II***)

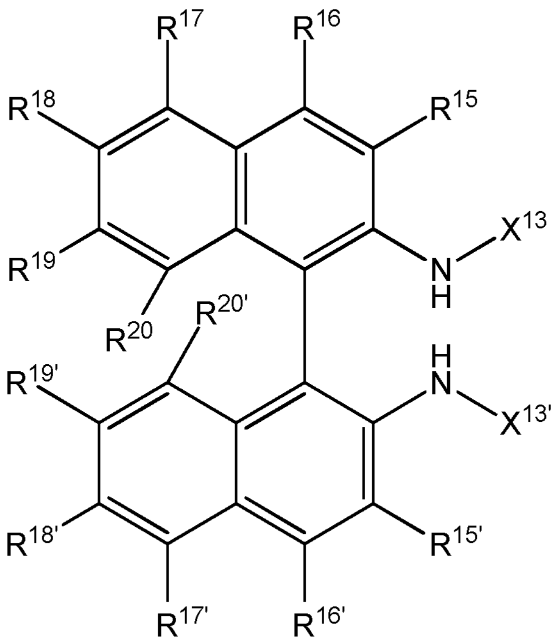

(**III***)

where

$R^{11}$, $R^{12}$, $R^{13}$, $R^{14}$, $R^{11'}$, $R^{12'}$, $R^{13'}$, $R^{14'}$ are selected from:
-H, - ($C_1$-$C_{12}$) -alkyl, -0- ($C_1$-$C_{12}$) -alkyl, -0- ($C_6$-$C_{20}$) -aryl, -($C_6$-$C_{20}$) -aryl, -S-alkyl, -S-aryl, halogen, -COO-($C_1$-$C_{12}$) -alkyl,-CONH- ($C_1$-$C_{12}$) -alkyl, -CO- ($C_1$-$C_{12}$) -alkyl, -CO- ($C_6$-$C_{20}$) -aryl,-COOH, -OH, -$SO_3$H, -CN, -N[($C_1$-$C_{12}$) -alkyl]$_2$;
$R^{15}$, $R^{16}$, $R^{17}$, $R^{18}$, $R^{19}$, $R^{20}$, $R^{15'}$, $R^{16'}$, $R^{17'}$, $R^{18'}$, $R^{19'}$, $R^{20'}$ are selected from:

-H, - ($C_1$-$C_{12}$) -alkyl, -0- ($C_1$-$C_{12}$) -alkyl, -0- ($C_6$-$C_{20}$) -aryl, -($C_6$-$C_{20}$) -aryl, -S-alkyl, -S-aryl, halogen, -COO-($C_1$-$C_{12}$)-alkyl,-CONH-($C_1$-$C_{12}$)-alkyl,-CO-($C_1$-$C_{12}$)-alkyl,-CO-($C_6$-$C_{20}$)-aryl,-COOH,-OH,-$SO_3$H, -N[($C_1$-$C_{12}$) -alkyl]$_2$;
where the alkyl and aryl groups mentioned may be substituted;

$X^{11}$ and $X^{11'}$ are selected from:

tert-butyloxycarbonyl, methyloxycarbonyl, benzyloxycarbonyl, phenyloxycarbonyl, acetyl, trifluoroacetyl, benzoyl, sulphonyl, sulphenyl,
and $X^{11}$ is not the same radical as $X^{11'}$;

$X^{12}$ and $X^{12'}$ are selected from:

tert-butyloxycarbonyl, methyloxycarbonyl, benzyloxycarbonyl, phenyloxycarbonyl, acetyl, trifluoroacetyl, benzoyl, sulphonyl, sulphenyl,
and $X^{12}$ is not the same radical as $X^{12'}$;

$X^{13}$ and $X^{13'}$ are selected from:

tert-butyloxycarbonyl, methyloxycarbonyl, benzyloxycarbonyl, phenyloxycarbonyl, acetyl, trifluoroacetyl, benzoyl, sulphonyl, sulphenyl,
and $X^{13}$ is not the same radical as $X^{13'}$.

**Revendications**

**1.** Procédé pour la préparation de 2,2'-diaminobiaryles, comprenant les étapes de procédé :

a) transformation d'un composé selon la formule (IVa) ou (Va) :

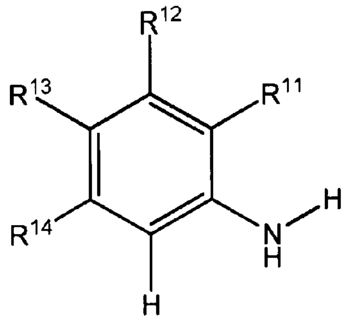

**(IVa)**

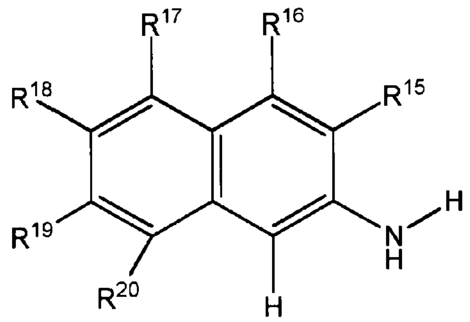

**(Va)**

(IVa) étant transformé en (IVb1) ou en (IVb2) ou
(Va) étant transformé en (Vb) :

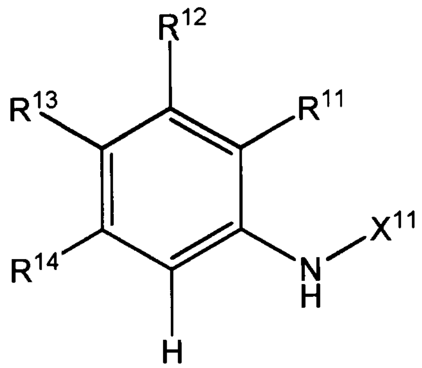

**(IVb1)**

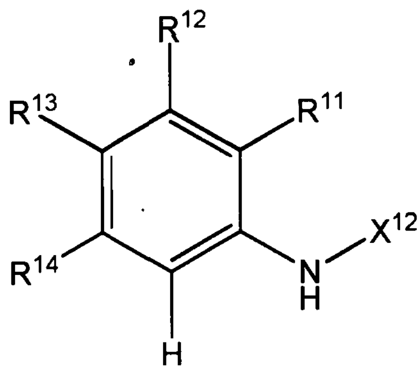

**(IVb2)**

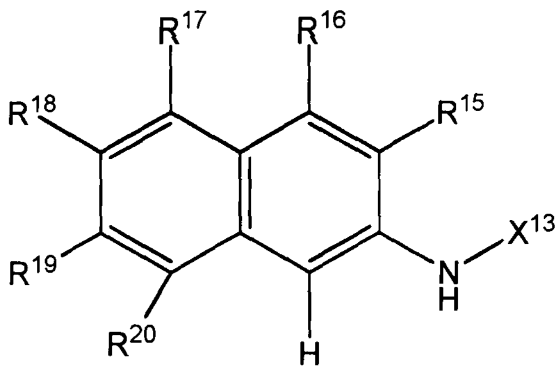

**(Vb)**

b) transformation d'un composé selon la formule (VIa) ou (VIIa) :

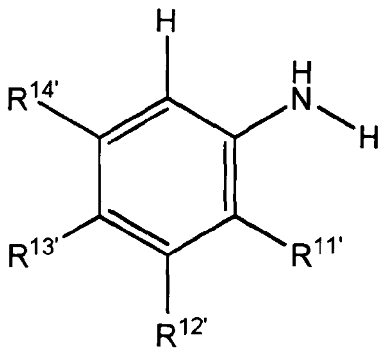

**(VIa)**

,

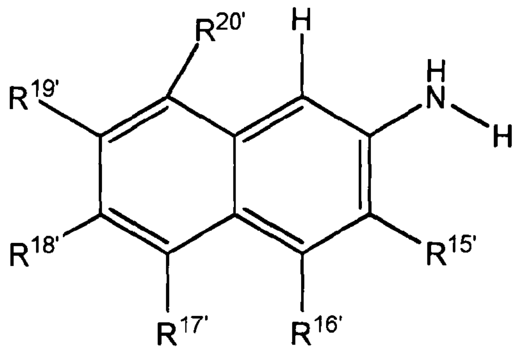

**(VIIa)**

(VIa) étant transformé en (VIb) ou
(VIIa) étant transformé en (VIIb1) ou en (VIIb2) :

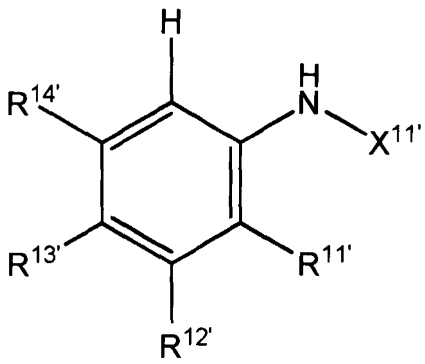

**(VIb)**

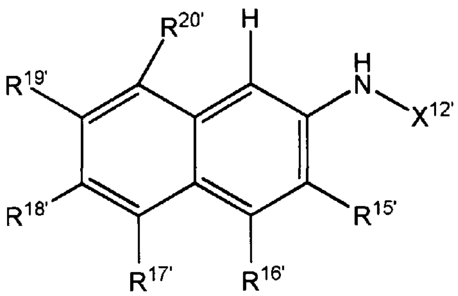

**(VIIb1)**

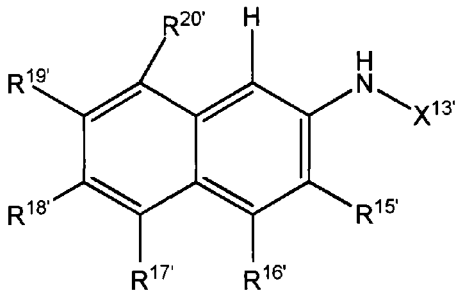

**(VIIb2)**

c) couplage électrochimique de :

- (IVb1) avec (VIb) en (I*) ; ou
- (IVb2) avec (VIIb1) en (II*) ; ou
- (Vb) avec (VIIb2) en (III*),

le composé présentant le potentiel d'oxydation le plus élevé étant à chaque fois utilisé en excès :

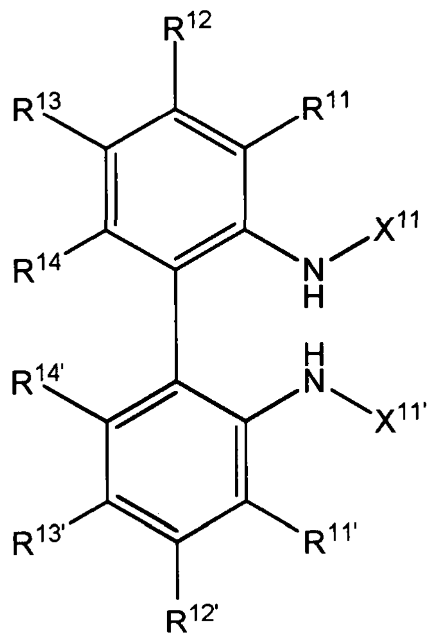

(I*)

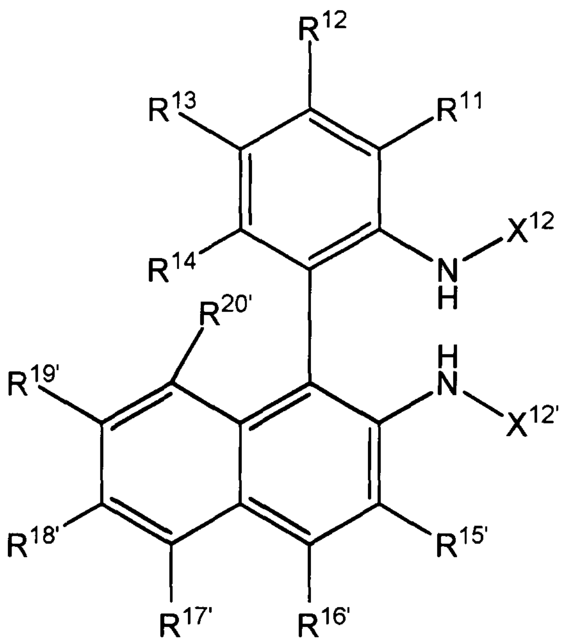

(II*)

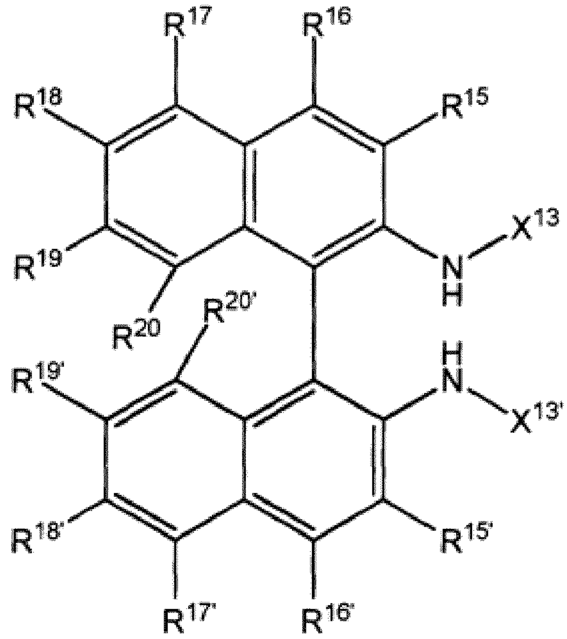

(III*)

dans lesquelles

$R^{11}$, $R^{12}$, $R^{13}$, $R^{14}$, $R^{11'}$, $R^{12'}$, $R^{13'}$, $R^{14'}$ sont choisis parmi : -H, -($C_1$-$C_{12}$)-alkyle, -O-($C_1$-$C_{12}$)-alkyle, -O-($C_6$-$C_{20}$)-aryle, -($C_6$-$C_{20}$) -aryle, -S-alkyle, -S-aryle, halogène, -COO-($C_1$-$C_{12}$)-alkyle, -CONH-($C_1$-$C_{12}$)-alkyle, -CO-($C_1$-$C_{12}$)-alkyle, -CO-($C_6$-$C_{20}$)-aryle, -COOH, -OH, -$SO_3$H, -CN, -N[($C_1$-$C_{12}$)-alkyle]$_2$ ;

$R^{15}$, $R^{16}$, $R^{17}$, $R^{18}$, $R^{19}$, $R^{20}$, $R^{15'}$, $R^{16'}$, $R^{17'}$, $R^{18'}$, $R^{19'}$, $R^{20'}$ sont choisis parmi :

-H, -($C_1$-$C_{12}$)-alkyle, -O-($C_1$-$C_{12}$)-alkyle, -O-($C_6$-$C_{20}$)-aryle, -($C_6$-$C_{20}$) -aryle, -S-alkyle, -S-aryle, halogène, -COO-($C_1$-$C_{12}$)-alkyle, -CONH-($C_1$-$C_{12}$)-alkyle, -CO-($C_1$-$C_{12}$)-alkyle, -CO-($C_6$-$C_{20}$)-aryle, -COOH, -OH, -$SO_3$H, -N[($C_1$-$C_{12}$)-alkyle]$_2$ ;

les groupes alkyle et aryle mentionnés pouvant être substitués ;

$X^{11}$ et $X^{11'}$ sont choisis parmi :

tert-butyloxycarbonyle, méthyloxycarbonyle, benzyloxycarbonyle, phényloxycarbonyle, acétyle, trifluoroacétyle, benzoyle, sulfonyle, sulfényle et

$X^{11}$ ne représente pas le même radical que $X^{11'}$ ;

$X^{12}$ et $X^{12'}$ sont choisis parmi :

tert-butyloxycarbonyle, méthyloxycarbonyle, benzyloxycarbonyle, phényloxycarbonyle, acétyle, trifluoroacétyle, benzoyle, sulfonyle, sulfényle et

$X^{12}$ ne représente pas le même radical que $X^{12'}$ ;

$X^{13}$ et $X^{13'}$ sont choisis parmi :

tert-butyloxycarbonyle, méthyloxycarbonyle, benzyloxycarbonyle, phényloxycarbonyle, acétyle, trifluoroacétyle, benzoyle, sulfonyle, sulfényle et

$X^{13}$ ne représente pas le même radical que $X^{13'}$.

Figur 1

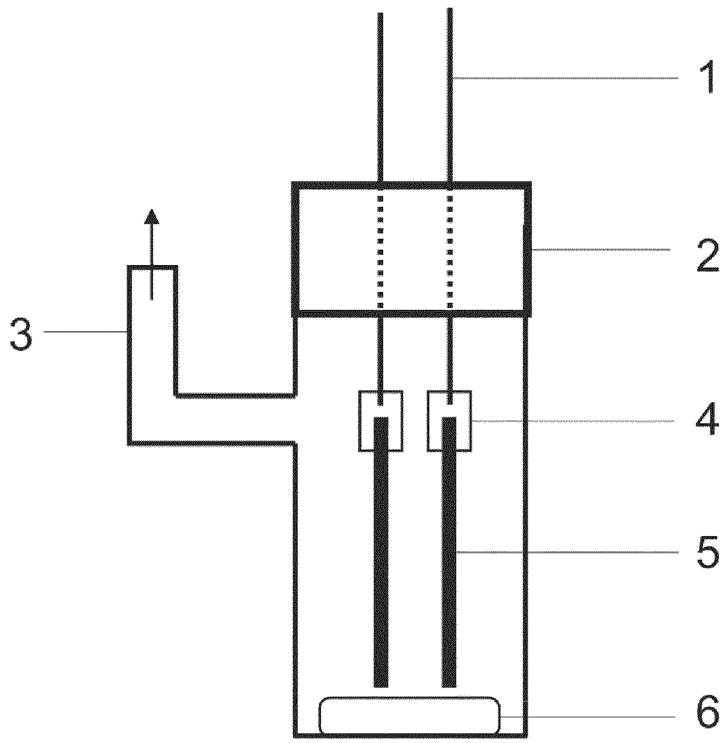

Figur 2

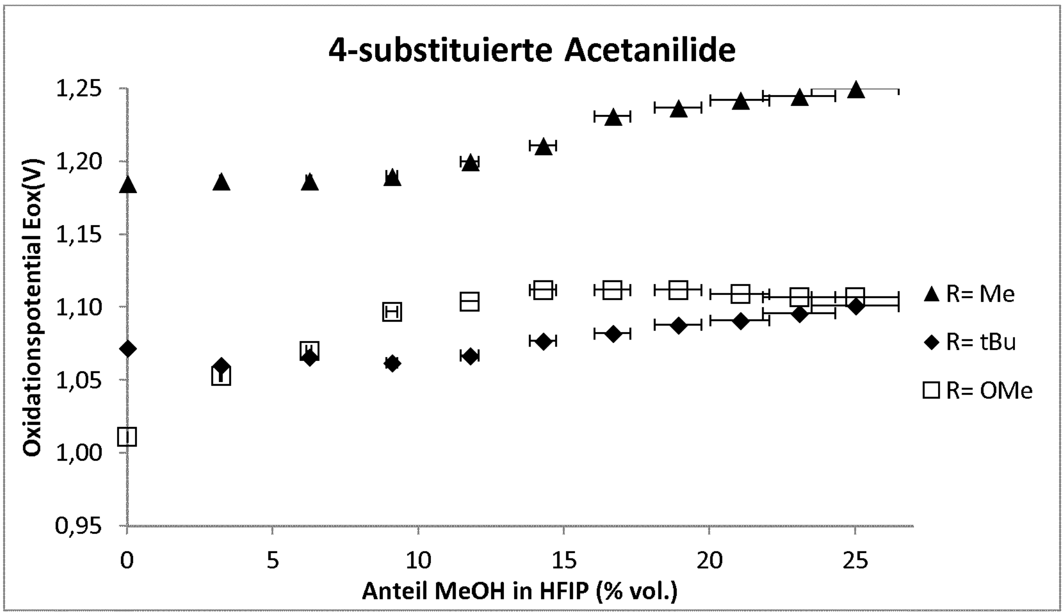


Figur 3

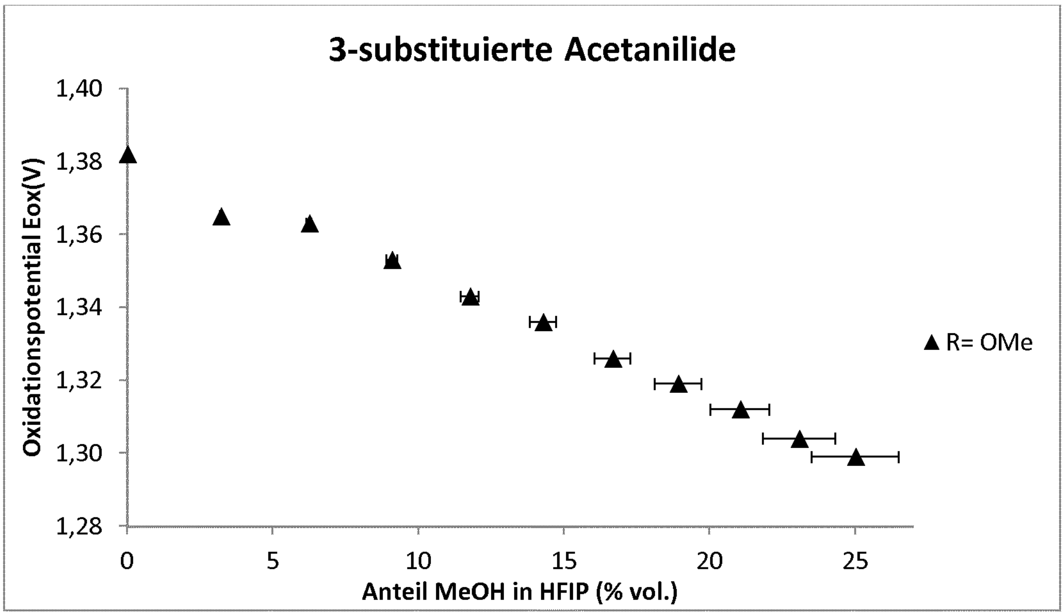

Figur 4

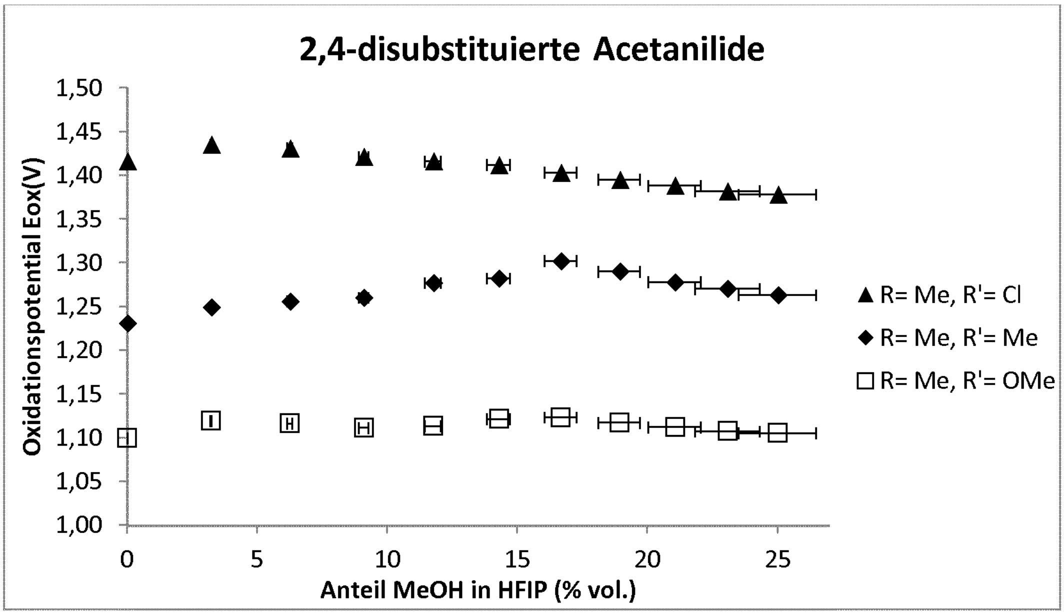


Figur 5

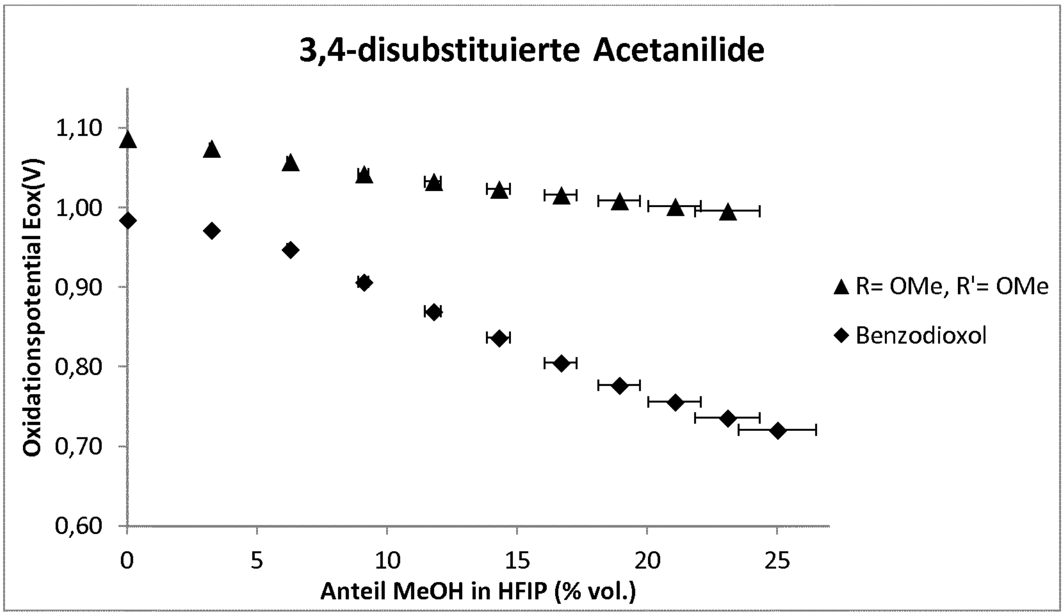

Figur 6

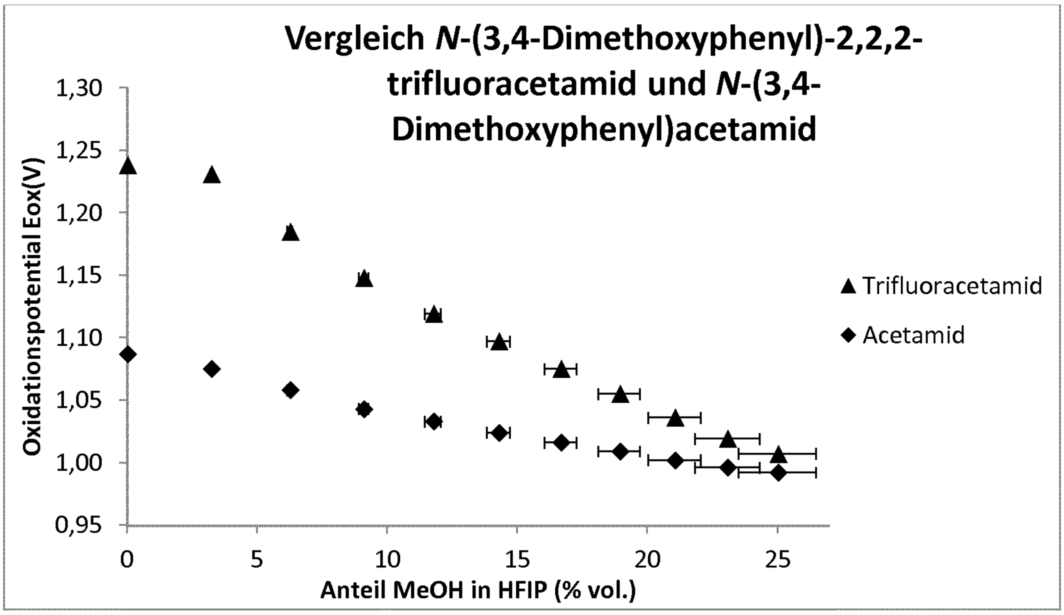


Figur 7

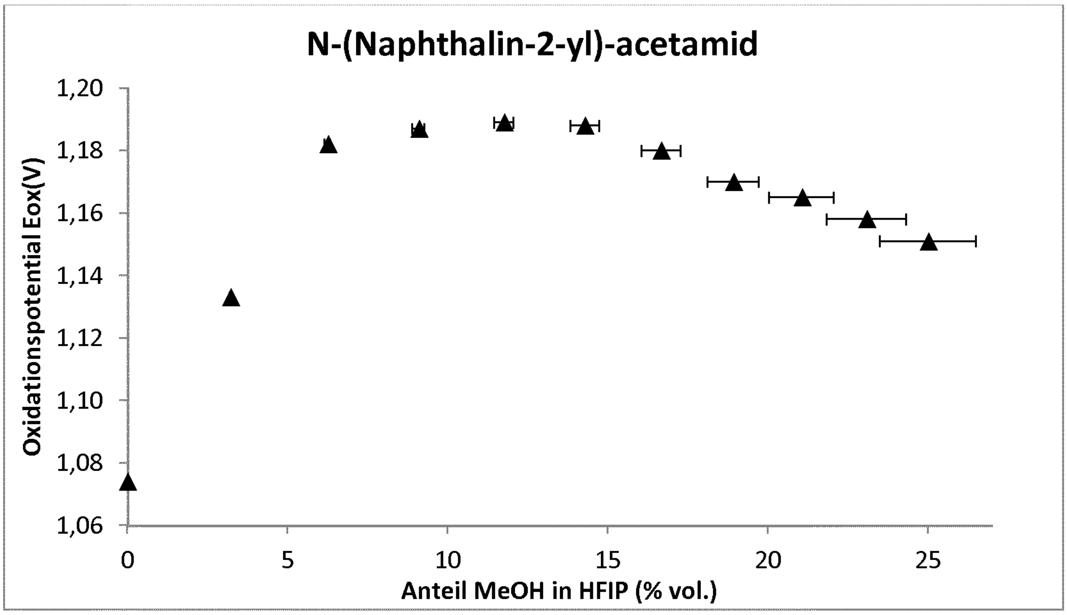

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **M. SMRCINA ; S. VYSKOCIL ; B. MACA ; M. POLASEK ; T. A. CLAXTON ; A. P. ABBOTT ; P. KOCOVSKY.** *J. Org. Chem.,* 1994, vol. 59, 2156-2163
- **J.-F. CUI ; H. HUANG ; H. WONG.** *Synlett,* 2011, vol. 7, 1018-1022
- **W. KALK ; H.-S. BIEN ; K.-H. SCHÜNDEHÜTTE.** *Justus Liebigs Ann. Chem.,* 1977, 329-337
- **S. ZHANG ; D. ZHANG ; L. S. LIEBESKIND.** *J. Org. Chem.,* 1997, vol. 62, 2312-2313
- **Y.-K. LIM ; J.-W. JUNG ; H. LEE ; C.-G. CHO.** *J. Org. Chem.,* 2004, vol. 69, 5778-5781
- **S.-E. SUH ; I.-K. PARK ; B.-Y. LIM ; C.-G. CHO.** *Eur. J. Org. Chem.,* 2011, vol. 3, 455-457
- **B.-Y. LIM ; M.-K. CHOI ; C.-G. CHO.** *Tetrahedron Letters,* 2011, vol. 52, 6015-6017
- **M. SHI ; W.-L. DUAN ; G.-B. RONG.** *Chirality,* 2004, vol. 16, 642-651
- **J. M. INSOLE.** *J.Chem. Soc. C,* 1971, 1712-1715
- **P. G. M. WUTS ; T. W. GREENE.** Greene's Protective Groups in Organic Synthesis. John Wiley and Sons, 2007